(19) Europäisches Patentamt
European Patent Office
Office européen des brevets

(11) EP 1 572 137 B1

(12) **FASCICULE DE BREVET EUROPEEN**

(45) Date de publication et mention de la délivrance du brevet:
**15.03.2017 Bulletin 2017/11**

(21) Numéro de dépôt: **03813190.0**

(22) Date de dépôt: **12.12.2003**

(51) Int Cl.:
***C08F 290/04*** (2006.01) ***C08F 290/06*** (2006.01)
***C08L 51/08*** (2006.01) ***C08L 51/00*** (2006.01)
***A61Q 1/06*** (2006.01) ***A61Q 1/10*** (2006.01)
***A61K 8/58*** (2006.01) ***A61Q 17/04*** (2006.01)
***A61Q 19/00*** (2006.01) ***A61Q 1/02*** (2006.01)
***A61Q 1/08*** (2006.01) ***A61Q 19/04*** (2006.01)
***A61K 8/893*** (2006.01) ***A61K 8/91*** (2006.01)
***A61K 8/04*** (2006.01) ***C08F 265/04*** (2006.01)
***C08F 265/00*** (2006.01) ***C08F 283/06*** (2006.01)

(86) Numéro de dépôt international:
**PCT/FR2003/003714**

(87) Numéro de publication internationale:
**WO 2004/055082 (01.07.2004 Gazette 2004/27)**

(54) **COMPOSITION COSMETIQUE COMPRENANT UNE DISPERSION DE PARTICULES D'UN POLYMERE ETHYLENIQUE GREFFE NON SILICONE DANS UNE PHASE GRASSE LIQUIDE**

KOSMETISCHE ZUSAMMENSETZUNG, DIE EINE DISPERSION VON TEILCHEN EINES SILIKONFREIEN GEPFROPFTEN ETHYLENPOLYMERS IN EINER FLÜSSIGEN ÖLPHASE ENTHÄLT

NON-TRANSFER COSMETIC COMPOSITION COMPRISING A DISPERSION OF PARTICLES OF A SILICONE-FREE GRAFTED ETHYLENE POLYMER IN A LIQUID FATTY PHASE

(84) Etats contractants désignés:
**AT BE BG CH CY CZ DE DK EE ES FI FR GB GR HU IE IT LI LU MC NL PT RO SE SI SK TR**

(30) Priorité: **12.12.2002 FR 0212737**
**12.12.2002 FR 0215738**
**12.12.2002 FR 0215739**

(43) Date de publication de la demande:
**14.09.2005 Bulletin 2005/37**

(73) Titulaire: **L'Oréal**
**75008 Paris (FR)**

(72) Inventeurs:
- **BLIN, Xavier**
**F-75015 Paris (FR)**
- **JAGER LEZER, Nathalie**
**F-91370 Verrières-le-Buisson (FR)**
- **LION, Bertrand**
**F-75012 Paris (FR)**

(74) Mandataire: **Kromer, Christophe et al**
**L'Oréal**
**Service DIPI**
**9 Rue Pierre Dreyfus**
**92110 Clichy (FR)**

(56) Documents cités:
**EP-A- 1 064 919**
**EP-A1- 0 923 928**
**WO-A-00/28948**
**FR-A- 2 746 640**
**US-A- 6 139 826**

EP 1 572 137 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention a pour objet une composition cosmétique comprenant une dispersion de particules d'un polymère éthylénique greffé non siliconé dans une phase grasse liquide, destinée à être appliquée sur les matières kératiniques d'êtres humains, comme la peau, les lèvres, les ongles, les fibres kératiniques tels que les cils, les sourcils, les cheveux.

La composition selon l'invention peut être une composition de maquillage ou une composition de soin des matières kératiniques, en particulier de la peau, des lèvres et des fibres kératiniques, notamment des cils, et de préférence une composition de maquillage.

La composition de maquillage peut être un produit de maquillage des lèvres (rouge à lèvres), un fond de teint, un fard à paupières, un fard à joue, un produit anticernes, un eye-liner, un produit de maquillage du corps, un mascara, un vernis à ongles, un produit de maquillage des cheveux.

La composition de soin peut être un produit de soin de la peau du corps et du visage, notamment un produit solaire, un produit de coloration de la peau (tel qu'un autobronzant). La composition peut être également un produit capillaire, notamment pour le maintien de la coiffure ou la mise en forme des cheveux ou encore un produit de soin des cils.

Les compositions de rouge à lèvres et fond de teint sont couramment employées pour apporter une couleur esthétique aux lèvres ou à la peau, notamment au visage. Ces produits de maquillage contiennent généralement des phases grasses telles que des cires et des huiles, des pigments et/ou charges et éventuellement des additifs comme des actifs cosmétiques ou dermatologiques.

Ces compositions, lorsqu'elles sont appliquées sur la peau, présentent l'inconvénient de transférer, c'est-à-dire de se déposer au moins en partie, en laissant des traces, sur certains supports avec lesquels elles peuvent être mises en contact et notamment un verre, une tasse, une cigarette, un vêtement ou la peau. Il s'ensuit une persistance médiocre du film appliqué, nécessitant de renouveler régulièrement l'application de la composition de fond de teint ou de rouge à lèvres. Par ailleurs, l'apparition de ces traces inacceptables notamment sur les cols de chemisier peut écarter certaines femmes de l'utilisation de ce type de maquillage.

On recherche donc des compositions de maquillage pour les lèvres et la peau dites « sans transfert » qui présentent l'avantage de former un dépôt qui ne se dépose pas, au moins en partie, sur les supports avec lesquels elles sont mises en contact (verre, vêtements, cigarette, tissus).

Pour limiter le transfert des compositions cosmétiques, il est connu d'employer des huiles volatiles , notamment à des teneurs supérieures à 40 % en poids. Ces huiles volatiles présentes en grande quantité rendent le produit de maquillage, notamment le rouge à lèvres, inconfortable pour l'utilisatrice : le dépôt de maquillage confère une sensation de dessèchement et de tiraillement.

Il est également connu des produits sous forme de deux compositions distinctes à appliquer l'une sur l'autre sur les lèvres pour obtenir un maquillage sans transfert. Par exemple, le produit LIP FINITY de MAX FACTOR est un produit bi-couche dont la composition de base (dite base coat) contient une résine de silicone et des huile volatiles, et la composition de surface (dite top coat) contient un ester de saccharose (comme décrit dans la demande WO 97/17057) pour améliorer le confort du produit de maquillage non transfert. Toutefois, l'application de deux compositions pour se maquiller peut être rédhibitoire pour certaines utilisatrices.

La demande EP-A-0923928 décrit une composition cosmétique comprenant un polymère dispersible dans une phase grasse liquide et dont les particules sont stabilisées un surface avec un polymère stabilisant.

La présente invention a pour but de fournir une nouvelle voie de formulation d'une composition cosmétique, notamment de maquillage, permettant d'obtenir un dépôt ayant de bonnes propriétés de non transfert, notamment sans utiliser un taux important d'huiles volatiles, et ce, sans requérir l'application de deux compositions distinctes pour l'obtention du résultat.

L'invention a également pour but de fournir une composition cosmétique, notamment de maquillage, permettant d'obtenir un dépôt sur la peau, les lèvres ou les fibres kératiniques confortable.

Les inventeurs ont découvert qu'il est possible d'obtenir une telle composition en utilisant une dispersion de particules d'un polymère éthylénique greffé non siliconé dans une phase grasse liquide telle que décrite ci-après. La composition permet d'obtenir un dépôt, notamment un maquillage des matières kératiniques, en particulier de la peau, des lèvres ou des fibres kératiniques, présentant de bonnes propriétés de non transfert. De plus, le dépôt obtenu sur la peau ou les lèvres ne provoque pas de sensation de dessèchement ou de tiraillement pour l'utilisatrice : le dépôt est donc confortable.

De façon plus précise, la présente invention a donc pour objet une composition cosmétique comprenant, dans un milieu cosmétiquement acceptable, au moins une dispersion de particules, de préférence solides, d'un polymère éthylénique greffé non siliconé dans une phase grasse liquide telle que décrite ci après, la composition étant notamment telle que définie ci-après.

Avantageusement, selon un premier mode de réalisation de la composition selon l'invention, le polymère est tel que lorsqu'il est dispersé en quantité suffisante dans la composition, cette dernière est apte à former un dépôt ayant

un transfert inférieur ou égal à35%.

Avantageusement, la composition selon l'invention est apte à former un dépôt ayant un transfert inférieur ou égal à 30 %, de préférence inférieur ou égal à 25 %, de préférence inférieur ou égal à 20 %, de préférence inférieur ou égal à 15 %, de préférence inférieur ou égal à 10 %, de préférence inférieur ou égal à 5 %.

L'invention a également pour objet un procédé cosmétique de maquillage ou de soin non thérapeutique des matières kératiniques, en particulier de la peau, des lèvres ou des fibres kératiniques, comprenant l'application sur les matières kératiniques, en particulier sur la peau ou les lèvres, d'une composition telle que définie précédemment.

L'invention a également pour objet l'utilisation d'une composition telle que définie précédemment pour obtenir un dépôt non transfert, notamment un maquillage, sur les matières kératiniques, en particulier sur la peau, les lèvres ou les fibres kératiniques.

L'invention a également pour objet l'utilisation d'une dispersion de particules d'un polymère éthylénique greffé non siliconé dans une phase grasse liquide telle que défini précédemment, dans une composition cosmétique comprenant un milieu liquide organique cosmétiquement acceptable, pour obtenir un dépôt, notamment un maquillage, non transfert sur les matières kératiniques, en particulier sur la peau, les lèvre ou les fibres kératiniques.

Le transfert du dépôt obtenu avec la composition selon l'invention est déterminé selon le protocole décrit ci-après.

On préchauffe un support (rectangle de 40 mm X 70 mm et d'épaisseur 3 mm) de mousse de polyéthylène adhésif sur une des faces ayant une densité de 33 kg/m3 (vendue sous la dénomination RE40X70EP3 de la société JOINT TECHNIQUE LYONNAIS IND) sur une plaque chauffante maintenue à la température de 40 °C pour que la surface du support soit maintenue à une température de 33 °C $\pm$ 1 °C.

Tout en laissant le support sur la plaque chauffante, on applique la composition sur toute la surface non adhésive du support en l'étalant à l'aide d'un pinceau pour obtenir un dépôt de la composition d'environ 15 $\mu$m puis on laisse sécher pendant 30 minutes.

Après séchage, le support est collé par sa face adhésive sur une enclume d'un diamètre de 20 mm et munie d'un pas de vis. L'ensemble support/dépôt est ensuite découpé à l'aide d'un emporte- pièce d'un diamètre de 18 mm. L'enclume est ensuite vissée sur une presse (STATIF MANUEL IMADA SV-2 de la société SOMECO) équipée d'un dynanomètre (IMADA DPS-20 de la société SOMECO).

Un papier blanc pour photocopieuse de 80g/m2 est placé sur le socle de la presse puis on presse l'ensemble support/dépôt sur le papier à une pression de 2,5 kg pendant 30 secondes. Après retrait de l'ensemble support/dépôt, une partie du dépôt a transféré sur le papier. On mesure alors la couleur du dépôt transféré sur le papier à l'aide d'un colorimètre MINOLTA CR300 , la couleur étant caractérisée par les paramètres colorimétriques L*, a*, b*. On détermine les paramètres colorimétriques $L^*_0$, $a^*_0$, $b^*_0$ de la couleur du papier nu utilisé.

On détermine alors la différence de couleur $\Delta$E1 entre la couleur du dépôt transféré par rapport à la couleur du papier nu par la relation suivante.

$$\Delta E1 = \sqrt{(L^* - L_o^*)^2 + (a^* - a_o^*)^2 + (b^* - b_o^*)^2}$$

Par ailleurs, on prépare une référence de transfert total en appliquant la composition directement sur un papier identique à celui utilisé précédemment, à la température ambiante (25 °C), en étalant la composition à l'aide d'un pinceau et pour obtenir un dépôt de la composition d'environ 15 $\mu$m puis on laisse sécher pendant 30 minutes à la température ambiante (25 °C). Après séchage, on mesure directement les paramètres colorimétriques L*', a*', b*' de la couleur du dépôt mis sur le papier, correspondant à la couleur de référence de transfert total. On détermine les paramètres colorimétriques $L^{*'}_0$, $a^{*'}_0$, $b^{*'}_0$ de la couleur du papier nu utilisé.

On détermine alors la différence de couleur $\Delta$E2 entre la couleur de référence de transfert total par rapport à la couleur du papier nu par la relation suivante.

$$\Delta E2 = \sqrt{(L^{*'} - L_o^{*'})^2 + (a^{*'} - a_o^{*'})^2 + (b^{*'} - b_o^{*'})^2}$$

Le transfert de la composition, exprimé en pourcentage, est égal au rapport :

$$100 \times \Delta E1 / \Delta E2$$

La mesure est effectuée sur 4 supports à la suite et la valeur de transfert correspond à la moyenne des 4 mesures obtenues avec les 4 supports.
La composition selon l'invention comprend une dispersion de particules de polymère éthylénique greffé non siliconé.
Par polymère "éthylénique", on entend un polymère obtenu par polymérisation de monomères comprenant une insaturation éthylénique.
Par polymère greffé non siliconé, on entend un polymère greffé contenant majoritairement un macromonomère carboné et contenant éventuellement au plus 7 % en poids, de préférence au plus 5 % en poids, voire est exempt de macromonomère siliconé. La dispersion de polymère éthylénique greffé non siliconé est notamment exempte de polymère stabilisant distinct dudit polymère greffé, tels que ceux décrits dans EP749747, et les particules de polymère éthylénique greffé ne sont donc pas stabilisées en surface par de tels polymères stabilisants additionnels. Le polymère greffé est donc dispersé dans la phase grasse liquide en l'absence de stabilisant additionnel en surface des particules.
Par polymère greffé, on entend un polymère ayant un squelette comprenant au moins une chaîne latérale pendante ou située en bout de chaîne, et de préférence pendante.
Avantageusement, le polymère éthylénique greffé non siliconé comprend un squelette éthylénique insoluble dans ladite phase grasse liquide, et des chaînes latérales liées de manière covalente audit squelette et solubles dans la phase grasse liquide.
Le polymère éthylénique greffé non siliconé est notamment un polymère non réticulé. En particulier, le polymère est obtenu par polymérisation de monomères comprenant un seul groupement polymérisable.
De préférence, le polymère éthylénique greffé non siliconé est un polymère filmogène. Par polymère "filmogène", on entend un polymère apte à former à lui seul ou en présence d'un agent auxiliaire de filmification, un film continu, notamment à l'oeil et au toucher, et adhérent sur un support, notamment sur les matières kératiniques.
Selon un mode de réalisation de l'invention, le polymère éthylénique greffé est un polymère acrylique greffé.
Le polymère éthylénique greffé non siliconé est notamment susceptible d'être obtenu par polymérisation radicalaire dans un milieu organique de polymérisation :

- d'au moins un monomère éthylénique, en particulier d'au moins un monomère acrylique et éventuellement d'au moins un monomère additionnel vinylique non acrylique, pour former ledit squelette insoluble ; et
- d'au moins un macromonomère comportant un groupe terminal polymérisable pour former les chaînes latérales, ledit macromonomère ayant une masse moléculaire moyenne en poids supérieure ou égale à 200 et la teneur en macromonomère polymérisé représentant de 0,05 à 20 % en poids du polymère.

La phase grasse liquide peut contenir le milieu organique de polymérisation.
Le milieu organique liquide de dispersion, correspondant au milieu dans lequel est fourni le polymère greffé, peut être identique au milieu de polymérisation.
Toutefois, le milieu de polymérisation peut être substitué en tout ou partie par un autre milieu organique liquide. Cet autre milieu organique liquide peut être ajouté, après polymérisation, au milieu de polymérisation. Ce dernier est ensuite évaporé en tout ou partie.
La phase grasse liquide peut contenir des composés liquides organiques autres que ceux présents dans le milieu de dispersion. Ces autres composés sont choisis de manière à ce que le polymère greffé reste à l'état de dispersion dans la phase grasse liquide.
Le milieu liquide organique de dispersion est présent dans la phase grasse liquide de la composition selon l'invention du fait de l'introduction dans la composition de la dispersion de polymère greffé obtenue.

**<u>La phase grasse liquide :</u>**

La phase grasse liquide comprend, de préférence majoritairement un ou plusieurs composés organiques liquides (ou huiles) tels que définis ci-après.
En particulier, la phase grasse liquide est une phase organique liquide non aqueuse et non miscible à l'eau à la température ambiante (25 °C).
On entend par "composé organique liquide" un composé non aqueux qui est à l'état liquide à la température ambiante (25 °C) et qui s'écoule donc de son propre poids.
On entend par "composé siliconé" un composé contenant au moins un atome de silicium.
La composition selon l'invention contient avantageusement une huile volatile telle que décrite ci-après.

Par huile volatile, on entend une huile susceptible de s'évaporer de la peau ou des lèvres en moins d'une heure, ayant notamment une pression de vapeur, à température ambiante et pression atmosphérique allant de $10^{-3}$ à 300 mm de Hg (0,13 Pa à 40 000 Pa).

L'huile volatile peut être siliconée ou non siliconée. Elle peut être notamment choisie parmi l'octaméthylcyclotétrasiloxane, le décaméthylcyclopentasiloxane, le dodécaméthylcyclohexasiloxane, l'heptaméthyl-hexyltrisiloxane, l'heptaméthyloctyltrisiloxane, l'octaméthyltrisiloxane, le décaméthyltétrasiloxane, l'isododécane, l'isodécane, l'isohexadécane, et leurs mélanges.

L'huile volatile est avantageusement présente en une teneur allant de 1 % à 70 % en poids, par rapport au poids total de la composition, de préférence allant de 5 % à 50 % en poids, et préférentiellement allant de 10 % à 35 % en poids.

La phase grasse liquide peut contenir une huile non volatile telle que décrite ci-après. L'huile non volatile est avantageusement présente en une teneur allant de 1% à 80 % en poids, par rapport au poids total de la composition, de préférence allant de 5 % à 60 % en poids, et préférentiellement allant de 10 % à 50 % en poids.

Parmi les composés organiques liquides ou huiles pouvant être présents dans le milieu organique liquide de dispersion, on peut citer :

- les composés organiques liquides, notamment non siliconés ou siliconés, ayant un paramètre de solubilité global selon l'espace de solubilité de Hansen inférieur ou égal à 18 $(MPa)^{1/2}$, de préférence inférieur ou égal à 17 $(MPa)^{1/2}$,
- les monoalcools ayant un paramètre de solubilité global selon l'espace de solubilité de Hansen inférieur ou égal à 20 $(MPa)^{1/2}$; et
- leurs mélanges.

Le paramètre de solubilité global $\delta$ selon l'espace de solubilité de Hansen est défini dans l'article « Solubility parameter values » de Eric A.Grulke de l'ouvrage « Polymer Handbook », 3éme édition, Chapitre VII, p.519-559 par la relation :

$$\delta = (d_D^2 + d_P^2 + d_H^2)^{1/2}$$

dans laquelle

- $d_D$ caractérise les forces de dispersion de LONDON issues de la formation de dipôles induits lors des chocs moléculaires,
- $d_P$ caractérise les forces d'intéractions de DEBYE entre dipôles permanents, et
- $d_H$ caractérise les forces d'intéractions spécifiques (type liaisons hydrogène, acide/base, donneur/accepteur, etc.).

La définition des solvants dans l'espace de solubilité selon Hansen est décrite dans l'article de C.M.Hansen « The three dimensional solubility parameters » J.Paint Technol. 39, 105 (1967).

Parmi les composés liquides organiques, notamment non siliconés ou siliconés, ayant un paramètre de solubilité global selon l'espace de solubilité de Hansen inférieur ou égal à 18 $(Mpa)^{1/2}$, on peut citer des corps gras liquides, notamment des huiles, qui peuvent être choisis parmi les huiles naturelles ou synthétiques, carbonées, hydrocarbonées, fluorées, siliconées, éventuellement ramifiées, seules ou en mélange.

Parmi ces huiles, on peut citer les huiles végétales formées par des esters d'acides gras et de polyols, en particulier les triglycérides, telles que l'huile de tournesol, de sésame ou de colza, ou les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle.

On peut également citer les alcanes linéaires, ramifiés et/ou cycliques éventuellement volatils et notamment des huiles de paraffine, de vaseline, ou le polyisobutylène hydrogéné, l'isododécane, ou encore les 'ISOPARS', les isoparaffines volatiles. On peut citer également les esters, les éthers, les cétones.

On peut encore citer les huiles siliconées telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines, et les huiles siliconées volatiles, notamment cycliques.

En particulier, on peut citer les huiles de silicone, éventuellement ramifiées, volatiles et/ou non volatiles.

Par huile volatile, on entend une huile susceptible de s'évaporer de la peau ou des lèvres en moins d'une

heure, ayant notamment une pression de vapeur, à température ambiante et pression atmosphérique allant de $10^{-3}$ à 300 mm de Hg (0,13 Pa à 40 000 Pa).

Comme huile siliconée volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone. En particulier, on peut citer l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Comme huile siliconée non volatile, on peut citer les polydialkylsiloxanes non volatils, tels que les polydiméthylsiloxanes (PDMS) non volatils; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényl triméthicones, les phényl diméthicones, les phényl triméthylsiloxy diphénylsiloxanes, les diphényl diméthicones, les diphényl méthyldiphényl trisiloxanes, les polyméthylphénylsiloxanes; les polysiloxanes modifiés par des acides gras (notamment en $C_8$-$C_{20}$), des alcools gras (notamment en $C_8$-$C_{20}$) ou des polyoxyalkylènes (notamment polyoxyéthyléne et/ou polyoxypropylène); les polysiloxanes aminées ; les polysiloxanes à groupement hydroxyles; les polysiloxanes fluorés comportant un groupement fluoré pendant ou en bout de chaîne siliconée ayant de 1 à 12 atomes de carbone dont tout ou partie des hydrogène sont substitués par des atomes de fluor ; et leurs mélanges.

On peut citer, en particulier, comme composés organiques liquides non siliconés ayant un paramètre de solubilité global selon l'espace de solubilité de Hansen inférieur ou égal à 18 $(Mpa)^{1/2}$:

- les esters linéaires, ramifiés ou cycliques, ayant au moins 6 atomes de carbone, notamment de 6 à 30 atomes de carbone;
- les éthers ayant au moins 6 atomes de carbone, notamment de 6 à 30 atomes de carbone ; et
- les cétones ayant au moins 6 atomes de carbone, notamment de 6 à 30 atomes de carbone.

Par monoalcools liquides ayant un paramètre de solubilité global selon l'espace de solubilité de Hansen inférieur ou égal à 20 $(MPa)^{1/2}$, on entend les monoalcools liquides gras aliphatiques ayant de 6 à 30 atomes de carbone, la chaîne hydrocarbonée ne comportant pas de groupement de substitution. Comme monoalcools selon l'invention, on peut citer l'alcool oléique, le décanol, l'octyldodécanol et l'alcool linoléique.

Milieu non siliconé

Selon un premier mode de réalisation de l'invention, la phase grasse liquide peut être une phase grasse liquide non siliconée.

On entend par "phase grasse liquide non siliconée" une phase grasse comprenant un ou plusieurs composés organiques liquides ou huiles non siliconé(e)s, tels que ceux cités précédemment, lesdits composés non siliconés étant présents majoritairement dans la phase grasse liquide, c'est-à-dire à au moins 50 % en poids, notamment de 50 à 100 % en poids, de préférence de 60 % à 100 % en poids (par exemple de 60 à 99 % en poids), ou encore de 65 % à 100 % en poids (par exemple de 65 à 95 % en poids), par rapport au poids total de la phase grasse liquide.

Les composés organiques liquides non siliconés peuvent être notamment choisis parmi :

- les composés organiques liquides non siliconés ayant un paramètre de solubilité global selon l'espace de solubilité de Hansen inférieur ou égal à 18 $(MPa)^{1/2}$,
- les monoalcools ayant un paramètre de solubilité global selon l'espace de solubilité de Hansen inférieur ou égal à 20 $(MPa)^{1/2}$; et
- leurs mélanges.

Ladite phase grasse liquide non siliconée peut donc éventuellement comprendre des composés organiques liquide ou huiles siliconé(e)s, tels que ceux cités précédemment, qui peuvent être présents en une quantité inférieure à 50 % en poids, notamment allant de 0,1 à 40 % en poids, voire allant de 1 à 35 % en poids, ou encore allant de 5 à 30 % en poids, par rapport au poids total de la phase grasse liquide.

Selon un mode particulier de réalisation de l'invention, la phase grasse liquide non siliconée ne contient pas de composés organiques liquides ou huiles siliconé(e)s.

Lorsque la phase grasse liquide est une phase grasse liquide non siliconée, les macromonères présents dans le polymère greffé sont avantageusement des macromonomères carbonés tels que décrits ci-après.

Milieu siliconé :

Selon un deuxième mode de réalisation de l'invention, la phase grasse liquide peut être une phase grasse

liquide siliconée.

On entend par "phase grasse liquide siliconée" une phase grasse comprenant un ou plusieurs composés organiques liquides siliconés ou huiles siliconées tels que ceux décrits précédemment, lesdits composés siliconés étant présents majoritairement dans la phase grasse liquide, c'est-à-dire à au moins 50 % en poids, notamment de 50 à 100 % en poids, de préférence de 60 % à 100 % en poids (par exemple de 60 à 99 % en poids), ou encore de 65 % à 100 % en poids (par exemple de 65 à 95 % en poids), par rapport au poids total de la phase grasse liquide.

Les composés organiques liquides siliconés peuvent être notamment choisis parmi :

- les composés organiques liquides, notamment non siliconés ou siliconés, ayant un paramètre de solubilité global selon l'espace de solubilité de Hansen inférieur ou égal à 18 $(MPa)^{1/2}$.

Ladite phase grasse liquide siliconée peut donc éventuellement comprendre des composés organiques liquides ou huiles non siliconé(e)s, tels que décrits précédemment, qui peuvent être présents en une quantité inférieure à 50 % en poids, notamment allant de 0,1 à 40 % en poids, voire allant de 1 à 35 % en poids, ou encore allant de 5 à 30 % en poids, par rapport au poids total da la phase grasse liquide.

Selon un mode particulier de réalisation de l'invention, la phase grasse liquide siliconée ne contient pas de composés organiques liquides non siliconés.

**Le polymère greffé :**

Le choix des monomères constituant le squelette du polymère, des macromonomères, le poids moléculaire du polymère, la proportion des monomères et des macromonomères peut être fait en fonction du milieu organique liquide de dispersion de manière à obtenir avantageusement une dispersion de particules de polymères greffés en particulier une dispersion stable, ce choix pouvant être effectué par l'homme du métier.

Par "dispersion stable", on entend une dispersion qui n'est pas susceptible de former de dépôt solide ou de déphasage liquide/solide notamment après une centrifugation, par exemple, à 4000 tours/minute pendant 15 minutes.

Le polymère éthylénique greffé non siliconé formant les particules en dispersion comprend donc un squelette insoluble dans ledit milieu de dispersion et une partie soluble dans ledit milieu de dispersion.

Le polymère éthylénique greffé peut être un polymère statistique.

Selon l'invention, on entend par "polymère éthylénique greffé" un polymère susceptible d'être obtenu par polymérisation radicalaire :

- d'un ou plusieurs monomère(s) éthylénique(s),
- avec un ou plusieurs macromonomère(s), dans un milieu organique de polymérisation.

Selon l'invention, on entend par "polymère acrylique greffé" un polymère susceptible d'être obtenu par polymérisation radicalaire :

- d'un ou plusieurs monomère(s) acrylique(s), et éventuellement d'un ou plusieurs monomère(s) additionnel(s) vinylique(s) non acrylique(s),
- avec un ou plusieurs macromonomère(s), dans un milieu organique de polymérisation.

Avantageusement, les monomères acryliques représentent de 50 à 100 % en poids, de préférence de 55 à 100 % en poids (notamment de 55 à 95 % en poids), préférentiellement de 60 à 100 % en poids (notamment de 60 à 90 % en poids) du mélange monomères acryliques + monomères vinyliques non acryliques éventuels.

De préférence, les monomères acryliques sont choisis parmi les monomères dont l'homopolymère est insoluble dans le milieu de dispersion considéré, c'est-à-dire que l'homopolymère est sous forme solide (ou non dissous) à une concentration supérieure ou égale à 5% en poids à température ambiante (20°C) dans ledit milieu de dispersion.

<u>Macromonomères :</u>

Selon l'invention, on entend par "macromonomère ayant un groupe terminal polymérisable" tout polymère comportant sur une seule de ses extrémités un groupe terminal polymérisable apte à réagir lors de la réaction de polymérisation avec les monomères acryliques et éventuellement les monomères vinyliques non acryliques additionnels constituant le squelette. Le macromonomère permet de former les chaînes latérales du polymère acrylique greffé. Le groupe polymérisable du macromonomère peut être avantageusement un groupe à insaturation éthylénique susceptible de se polymériser par voie radicalaire avec les monomères constituant le squelette.

Le macromère est un macromère carboné.

Par "macromonomère carboné" on entend un macromonomère non siliconé, et notamment un macromonomère oligomère obtenu par polymérisation de monomère(s) non siliconé(s) à insaturation éthylénique, et principalement par polymérisation de monomères acryliques et/ou vinyliques non acryliques.

Par "macromonomère siliconé" on entend un macromonomère organopolysiloxane, et en particulier un macromonomère polydiméthylsiloxane.

De préférence, le macromonomère est choisi parmi les macromonomères dont l'homopolymère est soluble dans le milieu de dispersion considéré, c'est-à-dire complètement dissous à une concentration supérieure ou égale à 5 % en poids et à température ambiante dans ledit milieu de dispersion.

Ainsi, le polymère acrylique greffé comprend un squelette (ou chaîne principale) constitué par un enchaînement de motifs acryliques résultant de la polymérisation notamment d'un ou plusieurs monomères acryliques et des chaînes latérales (ou greffons) issus de la réaction des macromonomères, lesdites chaînes latérales étant liées de manière covalente à ladite chaîne principale.

Le squelette (ou chaîne principale) est insoluble dans le milieu de dispersionconsidéré alors que les chaînes latérales (ou greffons) sont solubles dans ledit milieu de dispersion.

<u>Les monomères :</u>

Par "monomère acryliques", on entend dans la présente demande des monomères choisis parmi l'acide (méth)acrylique, les esters de l'acide (méth)acrylique (appelés également les (méth)acrylates), les amides de l'acide (méthacrylique) (appelés également les (méth)acrylamides).

Comme monomère acrylique susceptible d'être employé pour former le squelette insoluble du polymère, on peut citer, seul ou en mélange, les monomères suivants, ainsi que leurs sels :

- (i) les (méth)acrylates de formule :

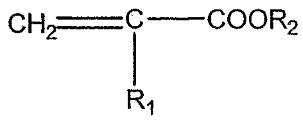

dans laquelle :

- $R_1$ désigne un atome d'hydrogène ou un groupe méthyle ;
- $R_2$ représente un groupe choisi parmi :
    - un groupe alkyle linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_4$; et/ou pouvant être substitué par au moins un groupe polyoxyalkylène, en particulier avec alkylène en $C_2$-$C_4$, notamment polyoxyéthylène et/ou polyoxypropylène, ledit groupe polyoxyalkylène étant constitué par la répétition de 5 à 30 motifs oxyalkylène;
    - un groupe alkyle cyclique comprenant de 3 à 6 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, Cl, Br, I);

A titre d'exemples de $R_2$, on peut citer le groupe méthyle, éthyle, propyle, butyle, isobutyle, méthoxyéthyle, éthoxyéthyle, méthoxy-polyoxyéthylène 350 OE , trifluoroéthyle, 2-hydroxyéthyle, 2-hydroxypropyle, diméthylaminoéthyle, diéthylaminoéthyle, diméthylaminopropyle.

- (ii) les (méth)acrylamides de formule :

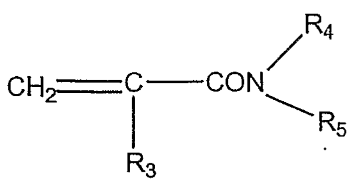

dans laquelle :

- $R_3$ désigne un atome d'hydrogène ou un groupe méthyle ;
- $R_4$ et $R_5$, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_4$;ou
- $R_4$ représente un atome d'hydrogène et $R_5$ représente un groupe 1,1-diméthyl-3-oxobutyle.

A titre d'exemples de groupes alkyles pouvant constituer $R_4$ et $R_5$, on peut citer n-butyle, t-butyle, n-propyle, diméthylaminoéthyle, diéthylaminoéthyle, diméthylaminopropyle.

- (iii) les monomères (méth)acryliques comprenant au moins une fonction acide carboxylique, phosphorique ou sulfonique, tels que l'acide acrylique, l'acide méthacrylique, l'acide acrylamidopropanesulfonique.

Parmi ces monomères acryliques, on peut tout particulièrement citer les (méth)acrylates de méthyle, d'éthyle, de propyle, de butyle, d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle ; le diméthylaminopropylméthacrylamide; et leurs sels ; et leurs mélanges.

De préférence, les monomères acryliques sont choisis parmi l'acrylate de méthyle, l'acrylate de méthoxyéthyle, le méthacrylate de méthyle, le méthacrylate de 2-hydroxyéthyle, l'acide (meth)acrylique, le méthacrylate de diméthylaminoéthyle, et leurs mélanges.

Parmi les monomères additionnels vinyliques non acryliques, on peut citer :

- les esters vinylique de formule : $R_6$-COO-CH=$CH_2$
  dans laquelle $R_6$ représente un groupe alkyle linéaire ou ramifié, comprenant de 1 à 6 atomes, ou un groupe alkyle cyclique comportant de 3 à 6 atomes de carbone et/ou un groupe aromatique, par exemple de type benzénique, anthracénique, et naphtalénique ;
- les monomères vinyliques non acryliques comprenant au moins une fonction acide carboxylique, phosphorique ou sulfonique, tels que l'acide crotonique, l'anhydride maléique, l'acide itaconique, l'acide fumarique, l'acide maléique, l'acide styrènesulfonique, l'acide vinylbenzoïque, l'acide vinylphosphorique, et leurs sels ;
- les monomères vinyliques non acryliques comprenant au moins une fonction amine tertiaire, tels que la 2-vinylpyridine, la 4-vinylpyridine ;
- et leurs mélanges.

Avantageusement, les monomères acryliques présents dans le polymère greffés comprennent au moins l'acide (méth)acrylique et au moins un monomère choisi parmi les (méth)acrylates et les (méth)acrylamides décrits précédemment aux points (i) et (ii). De préférence, les monomères acryliques comprennent au moins l'acide (méth)acrylique et au moins un monomère choisi parmi les (méth)acrylates d'alkyle en $C_1$-$C_3$. L'acide (méth)acrylique peut être présent en une teneur d'au moins 5 % en poids, par rapport au poids total du polymère, notamment allant de 5 % à 80 % en poids, de préférence d'au moins 10 % en poids, notamment allant de 10 % en poids à 70 % en poids, préférentiellement d'au moins 15 % en poids, notamment allant de 15 % à 60 % en poids.

Parmi les sels, on peut citer ceux obtenus par neutralisation des groupements acides à l'aide de base inorganiques telles que l'hydroxyde de sodium, l'hydroxyde de potassium, l'hydroxyde d'ammonium ou de bases organiques de type alkanols amines comme la monoéthanolamine, la diéthanolamine, la triéthanolamine, la 2-méthyl-2-amino-1-propanol.

On peut également citer les sels formés par neutralisation des motifs amine tertiaire, par exemple à l'aide d'acide minéral ou organique. Parmi les acides minéraux, on peut citer l'acide sulfurique ou l'acide chlorhydrique, l'acide bromhydrique, iodhydrique, l'acide phosphorique, l'acide borique. Parmi les acides organiques, on peut citer les acides comportant un ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide acétique ou l'acide propionique, l'acide téréphtalique, ainsi que l'acide citrique et l'acide tartrique.

Selon un mode de réalisation de l'invention, le polymère éthylénique greffé ne contient pas de monomères vinyliques non acryliques additionnels tels que décrits précédemment. Dans ce mode de réalisation, le squelette insoluble du polymère éthylénique greffé est formé uniquement de monomères acryliques tels que décrits précédemment.

Il est entendu que ces monomères acryliques non polymérisés peuvent être solubles dans le milieu de dispersion

considéré, mais le polymère formé avec ces monomères est insoluble dans le milieu de dispersion.

Comme monomère acrylique principal, on peut utiliser l'acrylate de méthyle, le méthacrylate de méthyle, l'acrylate d'éthyle, le méthacrylate d'éthyle, l'acrylate de n-propyle, le méthacrylate de n-propyle, l'acrylate d'iso-propyle et le méthacrylate d'isopropyle, et leurs mélanges.

On préfère tout particulièrement l'acrylate de méthyle, le méthacrylate de méthyle, le méthacrylate d'éthyle.

Les monomères acryliques additionnels peuvent être choisis parmi :

- l'acide (méth)acrylique et ses sels,
- les (méth)acrylates de formule (I) et leurs sels :

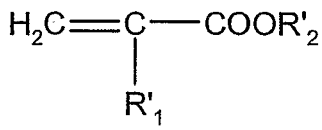

(I)

dans laquelle :

- $R'_1$ désigne un atome d'hydrogène ou un groupe méthyle ;
- $R'_2$ représente
    - un groupe alkyle linéaire ou ramifié comprenant de 1 à 6 atomes de carbone, ledit groupe comportant dans sa chaîne un ou plusieurs atomes d'oxygène et/ou comportant un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_3$ ;
    - un groupe alkyle cyclique comprenant de 3 à 6 atomes de carbone, ledit groupe pouvant comporter dans sa chaîne un ou plusieurs atomes d'oxygène et/ou pouvant comporter un ou plusieurs substituants choisis parmi OH et les atomes d'halogène (F, Cl, Br, I) ;

- et leurs mélanges.

A titre d'exemples de $R'_2$, on peut citer le groupe méthoxyéthyle, éthoxyéthyle, trifluoroéthyle; 2-hydroxyéthyle, 2-hydroxypropyle, diméthylaminoéthyle, diéthylaminoéthyle, diméthylaminopropyle.

Parmi ces monomères acryliques additionnels, on peut tout particulièrement citer l'acide (méth)acrylique, les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle, leurs sels, et leurs mélanges.

On préfère tout particulièrement l'acide acrylique, l'acide méthylacrylique.

Les macromonomères comportent à une des extrémités de la chaîne un groupe terminal polymérisable apte à réagir au cours de la polymérisation avec les monomères acryliques et éventuellement les monomères vinyliques additionnels, pour former les chaînes latérales du polymère éthylénique greffé. Ledit groupe terminal polymérisable peut être en particulier un groupe vinyle ou (méth)acrylate (ou (méth)acryloxy), et de préférence un groupe (méth)acrylate.

Les macromonomères sont choisis préférentiellement parmi les macromonomères dont l'homopolymère a une température de transition vitreuse (Tg) inférieure ou égale à 25°C, notamment allant de - 100°C à 25°C, de préférence allant de - 80°C à 0°C.

Les macromonomères ont une masse moléculaire moyenne en poids supérieure ou égale à 200, de préférence supérieure ou égale à 300, préférentiellement supérieure ou égale à 500, et plus préférentiellement supérieure à 600. De préférence, les macromonomères ont une masse moléculaire moyenne en poids (Mw) allant de 200 à 100 000, de préférence allant de 500 à 50 000, préférentiellement allant de 800 à 20 000, plus préférentiellement allant de 800 à 10000, et encore plus préférentiellement allant de 800 à 6000.

Dans la présente demande, les masses molaires moyennes en poids (Mw) et en nombre (Mn) sont déterminées par chromatographie liquide par perméation de gel (solvant THF, courbe d'étalonnage établie avec des étalons de polystyrène linéaire, détecteur réfractométrique).

Comme macromonomères carbonés, on peut en particulier citer :

- (i) les homopolymères et les copolymères (méth)acrylate d'alkyle linéaire ou ramifié en C8-C22, présentant un groupe terminal polymérisable choisi parmi les groupes vinyle ou (méth)acrylate parmi lesquels on peut citer en particulier : les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate ;

les macromonomères de poly(acrylate de stéaryle) ou de poly (méthacrylate de stéaryle) à extrémité mono(méth)acrylate.

De tels macromonomères sont notamment décrits dans les brevets EP895467 et EP96459 et dans l'article Gillman K.F., Polymer Letters, Vol 5, page 477-481 (1967).

On peut en particulier citer les macromonomères à base de poly(acrylate d'éthyl-2-hexyle) ou de poly(acrylate de dodécyle) à extrémité mono(méth)acrylate.

- (ii) les polyoléfines ayant un groupe terminal à insaturation éthylénique , en particulier ayant un groupement terminal (méth)acrylate. Comme exemple de telles polyoléfines, on peut citer en particulier les macromonomères suivants, étant entendu qu'ils ont un groupe terminal (méth)acrylate : les macromonomères de polyéthylène, les macromonomères de polypropylène, les macromonomères de copolymère polyéthylène/polypropylène, les macromonomères de copolymère polyéthylène/polybutylène, les macromonomères de polyisobutylène ; les macromonomères de polybutadiène; les macromonomères de polyisoprène ; les macromonomères de polybutadiène; les macromonomères de poly(éthylène/butylène)-polyisoprène ;

De tels macromonomères sont en particulier décrits dans US5625005 qui mentionne des macromonomères éthylène/butylène et éthylène/propylène à groupement terminal réactif (méth)acrylate.

On peut en particulier citer le méthacrylate de poly(éthylène/butylène), tel que celui commercialisé sous la dénomination Kraton Liquid L-1253 par Kraton Polymers.

De préférence, le macromonomère polymérisé (constituant les chaînes latérales du polymère greffé) représente de 0,1 à 15 % en poids du poids total du polymère, préférentiellement de 0,2 à 10 % en poids, et plus préférentiellement de 0,3 à 8 % en poids.

Comme polymère éthylénique greffé particulièrement préféré dispersé dans une phase grasse liquide non siliconée, on peut utiliser ceux obtenus par polymérisation :

- de l'acrylate de méthyle et du macromonomère polyéthylène/polybutylène à groupement terminal méthacrylate (notamment Kraton L-1253), en particulier dans un solvant choisi parmi l'isododécane, l'isononanoate d'isononyle, l'octyldodécanol , le malate de diisostéaryle, un benzoate d'alkyl $C_{12}$-$C_{15}$ (tel que Finsolv TN) ;
- de l'acrylate de méthoxyéthyle et du macromonomère polyéthylène/polybutylène à groupement terminal méthacrylate (notamment Kraton L-1253), en particulier dans l'isododécane ;
- des monomères acrylate de méthyle / méthacrylate de méthyle et du macromonomère polyéthylène/polybutylène à groupement terminal méthacrylate (notamment Kraton L-1253), en particulier dans l'isododécane ;
- des monomères acrylate de méthyle / acide acrylique et du macromonomère polyéthylène/polybutylène à groupement terminal méthacrylate (notamment Kraton L-1253), en particulier dans l'isododécane ;
- des monomères acrylate de méthyle / méthacrylate de diméthylaminoéthyle et du macromonomère polyéthylène/polybutylène à groupement terminal méthacrylate (notamment Kraton L-1253), en particulier dans l'isododécane ;
- des monomères acrylate de méthyle / méthacrylate de 2-hydroxyéthyle et du macromonomère polyéthylène/polybutylène à groupement terminal méthacrylate (notamment Kraton L-1253), en particulier dans l'isododécane.

De préférence, le polymère greffé a une masse moléculaire moyenne en poids (Mw) comprise entre 10 000 et 300 000, notamment entre 20 000 et 200 000, mieux encore entre 25 000 et 150 000.

Grâce aux caractéristiques susmentionnées, dans un milieu organique de dispersion donné, les polymères ont la capacité de se replier sur eux-mêmes, formant ainsi des particules de forme sensiblement sphérique, avec sur le pourtour de ces particules les chaînes latérales déployées, qui assurent la stabilité de ces particules. De telles particules résultant des caractéristiques du polymère greffé ont la particularité de ne pas s'agglomérer dans ledit milieu et donc de s'autostabiliser et de former une dispersion de particules de polymère particulièrement stable.

En particulier, les polymères éthyléniques greffés de la dispersion peuvent former des particules nanométriques, de taille moyenne allant de 10 à 400 nm, de préférence de 20 à 200 nm.

Du fait de cette taille très faible, les particules de polymère greffé en dispersion sont particulièrement stables et donc peu susceptibles de former des agglomérats.

La dispersion de polymère greffé peut donc être une dispersion stable et ne forme pas de sédiments, lorsqu'elle est placée pendant une durée prolongée (par exemple 24 heures) à température ambiante (25 °C).

De préférence, la dispersion de particules de polymère greffé présente un taux de matière sèche (ou extrait sec) en polymère pouvant aller de 40 % à 70 % en poids de matière sèche, notamment allant de 45 % à 65 % en poids.

On peut préparer la dispersion de particules de polymère greffé par un procédé comprenant une étape de copolymérisation radicalaire, dans un milieu organique de polymérisation, d'un ou plusieurs monomères acryliques tels que définis précédemment avec un ou plusieurs macromonomères tels que définis précédemment.

Comme indiqué précédemment, le milieu organique liquide de dispersion peut être identique ou different du milieu de polymérisation.

D'une manière classique, la copolymérisation peut être effectuée en présence d'un initiateur de polymérisation. Les initiateurs de polymérisation peuvent être des amorceurs radicalaires. De manière générale, un tel initiateur de polymérisation peut être choisi parmi les composés organiques peroxydés tels que le dilauroyl peroxyde, le dibenzoyl peroxyde, le tert-butyl peroxy-2-éthylhexanoate ; les composés diazotés tels que l'azobisisobutyronitrile, l'azobisdiméthylvalero-nitrile.

La réaction peut être également initiée à l'aide de photoinitiateurs ou par une radiation telle que des UV, des neutrons ou par plasma.

D'une manière générale, pour mettre en oeuvre ce procédé, on introduit, dans un réacteur de taille appropriée à la quantité de polymère que l'on va réaliser, au moins une partie du milieu organique de polymérisation, une partie des monomères acryliques et/ou vinyliques additionnels, qui constituera, après polymérisation, le squelette insoluble, la totalité du macromonomère (qui constituera les chaînes latérales du polymère) et une partie de l'initiateur de polymérisation. A ce stade d'introduction, le milieu réactionnel forme un milieu relativement homogène.

Le milieu réactionnel est ensuite agité et chauffé jusqu'à une température pour obtenir une polymérisation des monomères et macromonomères. Après un certain temps, le milieu initialement homogène et limpide conduit à une dispersion d'aspect laiteux. On ajoute ensuite un mélange constitué de la partie restante de monomères et de l'initiateur de polymérisation. Après un temps adéquat pendant lequel le mélange est chauffé sous agitation, le milieu se stabilise sous forme d'une dispersion laiteuse, la dispersion comprenant des particules de polymères stabilisés dans le milieu dans lequel elles ont été créées, ladite stabilisation étant due à la présence, dans le polymère, de chaînes latérales solubles dans ledit milieu de dispersion.

Le polymère greffé peut être présent dans la composition selon l'invention en une teneur en matière sèche (ou matière active) allant de 1 à 70% en poids par rapport au poids total de la composition, mieux de 5 à 60% en poids, de préférence allant de 6 à 45% et mieux allant de 8 à 40% en poids.

La composition selon l'invention peut également comprendre au moins corps gras solides à température ambiante notamment choisi parmi les cires, les corps gras pâteux, les gommes et leurs mélanges. Ces corps gras peuvent être d'origine animale, végétale, minérale ou synthétique.

Cire

La composition sel8on l'invention peut comprendre une cire ou un mélange de cires.

La cire considérée dans le cadre de la présente invention est d'une manière générale un composé lipophile, solide à température ambiante (25 °C), à changement d'état solide/liquide réversible, ayant un point de fusion supérieur ou égal à 30 °C pouvant aller jusqu'à 120 °C.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

En particulier, les cires convenant à l'invention peuvent présenter un point de fusion supérieur à 45° environ, et en particulier supérieur à 55 °C.

Le point de fusion de la cire peut être mesuré à l'aide d'un calorimètre à balayage différentiel (D.S.C.), par exemple le calorimètre vendu sous la dénomination DSC 30 par la société METLER.

Le protocole de mesure est le suivant :

Un échantillon de 15 mg de produit disposé dans un creuset est soumis à une première montée en température allant de 0 °C à 120 °C, à la vitesse de chauffe de 10 °C/ minute, puis est refroidi de 120 °C à 0 °C à une vitesse de refroidissement de 10 °C/minute et enfin soumis à une deuxième montée en température allant de 0 °C à 120 °C à une vitesse de chauffe de 5 °C/minute. Pendant la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides et rigides à température ambiante, d'origine animale, végétale, minérale ou de synthèse et leurs mélanges.

La cire peut également présenter une dureté allant de 0,05 MPa à 30 MPa, et de préférence allant de 6 MPa à 15 MPa . La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Le protocole de mesure est le suivant :

La cire est fondue à une température égale au point de fusion de la cire + 20 °C. La cire fondue est coulée dans

un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression maximale mesurée divisée par la surface du cylindre du texturomètre en contact avec la cire.

On peut notamment utiliser les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.

On peut aussi citer les cires obtenues par hydrogénation catalytique d'huiles animales ou végétales ayant des chaînes grasses, linéaires ou ramifiées, en C8-C32.

Parmi celles-ci, on peut notamment citer l'huile de jojoba hydrogénée, l'huile de jojoba isomérisée telle que l'huile de jojoba partiellement hydrogénée isomérisée trans fabriquée ou commercialisée par la société Desert Whale sous la référence commerciale ISO-JOJOBA-50®, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination « HEST 2T-4S » par la société HETERENE, le tétrabéhénate de di-(triméthylol-1,1,1 propane) vendue sous la dénomination HEST 2T-4B par la société HETERENE.

On peut encore citer les cires de silicone comme les alkyl ou alkoxy-diméticone ayant de 16 à 45 atomes de carbone, les cires fluorées.

On peut également utiliser la cire obtenue par hydrogénation d'huile d'olive estérifiée avec l'alcool stéarylique vendue sous la dénomination « PHYTOWAX Olive 18 L 57 » ou bien encore les cires obtenues par hydrogénation d'huile de ricin estérifiée avec l'alcool cétylique vendus sous la dénomination « PHYTOWAX ricin 16L64 et 22L73 », par la société SOPHIM. De telles cires sont décrites dans la demande FR-A-2792190.

Selon un mode de réalisation particulier, les compositions selon l'invention peuvent comprendre au moins une cire dite cire collante c'est-à-dire possédant un collant supérieur ou égal à 0,7 N.s et une dureté inférieure ou égale à 3,5 MPa.

L'utilisation d'une cire collante peut notamment permettre l'obtention d'une composition cosmétique qui s'applique facilement sur les fibres kératiniques, ayant une bonne accroche sur les fibres kératiniques et qui conduit à la formation d'un maquillage lisse, homogène et épaississant.

La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s à 10 N.s, et en particulier allant de 2 N.s à 5 N.s.

Le collant de la cire est déterminé par la mesure de l'évolution de la force (force de compression ou force d'étirement) en fonction du temps, à 20 °C à l'aide du texturomètre vendu sous la dénomination « TA-TX2i® » par la société RHEO, équipé d'un mobile en polymère acrylique en forme de cône formant un angle de 45°.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.

Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.

La dureté est mesurée selon le protocole décrit précédemment.

Comme cire collante, on peut utiliser un (hydroxystéaryloxy)stéarate d'alkyle en $C_{20}$-$C_{40}$ (le groupe alkyle comprenant de 20 à 40 atomes de carbone), seul ou en mélange, en particulier un 12-(12'-hydroxystéaryloxy)stéarate d'alkyle en $C_{20}$-$C_{40}$, de formule (II) :

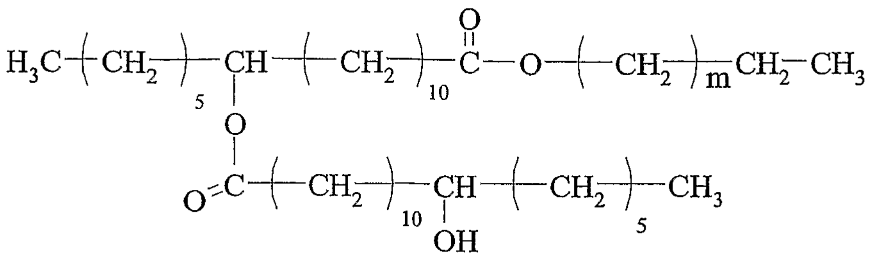

(II)

dans laquelle m est un entier allant de 18 à 38, ou un mélange de composés de formule (II).

Une telle cire est notamment vendue sous les dénominations « Kester Wax K 82 P® » et « Kester Wax K 80 P® » par la société KOSTER KEUNEN.

Les cires citées ci-dessus présentent généralement un point de fusion commençante inférieur à 45 °C.

La ou les cires peu(ven)t être présente(s) sous forme d'une microdispersion aqueuse de cire. On entend par microdispersion aqueuse de cire, une dispersion aqueuse de particules de cire, dans laquelle la taille desdites particules de cire est inférieure ou égale à environ 1 μm.

Les microdispersions de cire sont des dispersions stables de particules colloïdales de cire, et sont notamment décrites dans "Microemulsions Theory and Practice", L.M. Prince Ed., Academic Press (1977) pages 21-32.

En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.

Les microdispersion de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 μm (notamment allant de 0,02 μm à 0,99 μm), de préférence inférieures à 0,5 μm (notamment allant de 0,06 μm à 0,5 μm).

Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

Par corps gras pâteux, on entend un composé gras lipophile comportant à la température de 23°C une fraction liquide et une fraction solide.

Ledit composé pâteux a de préférence une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1 mm/s puis à une vitesse de mesure de 0,1 mm/s, la profondeur de pénétration étant de 0,3 mm. La valeur relevée de la dureté est celle du pic maximum.

La fraction liquide du composé pâteux mesurée à 23°C représente de préférence 9 à 97% en poids du composé. Cette fraction liquide à 23°C représente de préférence entre 15 et 85%, de préférence encore entre 40 et 85% en poids. La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée eu J/g. L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 80 à 100%, de préférence encore de 90 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0,1 à 50 % en poids de cires, par rapport au poids total de la composition, mieux de 1 à 40 % et encore mieux de 5 à 30% en poids.

La composition peut comprendre, outre le polymère en dispersion dans la phase grasse liquide décrit précédemment selon l'invention, un polymère additionnel tel qu'un polymère filmogène . Selon la présente invention, on entend par "polymère filmogène", un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle et leurs mélanges. Comme polymère filmogène, on peut citer en particulier les polymères acryliques, les polyuréthanes, les polyesters, les polyamides, les polyurées, les polymères cellulosiques comme la nitrocellulose.

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre. Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.. On peut également utiliser les pigments interférentiels, notamment à cristaux liquides ou multicouches.

En particulier, il a été constaté que l'utilisation de la dispersion de particules d'un polymère éthylénique greffé non siliconé dans une phase grasse liquide, telle que décrite précédemment, dans une composition cosmétique comprenant des matières colorantes pulvérulentes, en particulier des pigments, permet une bonne dispersion desdits pigments (sans sédimentation) et donc une bonne stabilité et une bonne homogénéité de la couleur dans le temps de la composition selon l'invention.

C'est pourquoi un objet de la présente invention est une composition cosmétique comprenant, dans un milieu cosmétiquement acceptable, une dispersion de particules d'un polymère éthylénique greffé non siliconé dans une phase grasse liquide et une matière colorante pulvérulente, notamment sous forme de pigments.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

La composition selon l'invention peut comprendre au moins une charge, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges

servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques expansées telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut également contenir des ingrédients couramment utilisés en cosmétique, tels que les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les fibres, les agents anti-chutes des cheveux, les agents de soin du cil, les agents antipelliculaires, les agents propulseurs, ou leurs mélanges.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, de préférence de 5 à 500, et mieux de 5 à 150.

En particulier, les fibres ont une longueur allant de 1 μm à 10 mm, de préférence de 0,1 mm à 5 mm et mieux de 0,3 mm à 3mm.

Les fibres utilisables dans la composition de l'invention peuvent être choisies parmi les fibres rigides ou non rigides, elles peuvent être d'origine synthétique ou naturelle, minérale ou organique.

A titre de fibres utilisables dans la composition selon l'invention, on peut citer les fibres non rigides telles que les fibres de polyamide (Nylon®) ou les fibres rigides telles que les fibres de polyimide-amide comme celles vendues sous les dénomination "KERMEL", " KERMEL TECH" par la société RHODIA ou de poly-(p-phénylène-téréphtalamide) (ou d'aramide) notamment vendues sous la dénomination Kevlar ® par la société DUPONT DE NEMOURS.

Les fibres peuvent être présentes dans la composition selon l'invention en une teneur allant de 0,1 à 10% en poids, mieux de 0,5 à 5% en poids par rapport au poids total de la composition.

Les gélifiants utilisables dans les compositions selon l'invention peuvent être des gélifiant hydrophiles, lipophiles, organiques ou minéraux, polymériques ou moléculaires.

Comme gélifiant lipophile minéral, on peut citer les argiles éventuellement modifiées comme les hectorites modifiées par un chlorure d'ammonium d'acide gras en $C_{10}$ à $C_{22}$, comme l'hectorite modifiée par du chlorure de di-stéaryl di-méthyl ammonium telle que, par exemple, celle commercialisée sous la dénomination de « Bentone 38V® » par la société ELEMENTIS.

On peut également citer la silice pyrogénée éventuellement traitée hydrophobe en surface dont la taille des particules est inférieure à 1 μm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :

- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (6ème édition, 1995). Elles sont par exemple commercialisées sous les références « Aerosil R812® » par la société DEGUSSA, « CAB-O-SIL TS-530®» par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (6ème édition, 1995). Elles sont par exemple commercialisées sous les références « Aerosil R972® », et « Aerosil R974® » par la société DEGUSSA, « CAB-O-SIL TS-610® » et « CAB-O-SIL TS-720®» par la société CABOT.

La silice pyrogénée hydrophobe présente en particulier une taille de particules pouvant être nanométrique à micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les dénominations de « KSG6® », « KSG16® » et de « KSG18® » par la société SHIN-ETSU, de « Trefil E-505C® » et « Trefil E-506C® » par la société DOW-CORNING, de « Gransil SR-CYC® », « SR DMF10® », « SR-DC556® », « SR 5CYC gel® »,

« SR DMF 10 gel® » et de « SR DC 556 gel® » par la société GRANT INDUSTRIES, de « SF 1204® » et de « JK 113® » par la société GENERAL ELECTRIC ; l'éthylcellulose comme celle vendue sous la dénomination d'« Ethocel® » par la société DOW CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en $C_1$ à $C_6$, et en particulier en $C_1$ à $C_3$ et leurs mélanges. Les copolymères séquencés de type « dibloc » ou « tribloc » du type polystyrène/polyisoprène, polystyrène/polybutadiène tels que ceux commercialisés sous la dénomination de « Luvitol HSB® » par la société BASF, du type polystyrène/copoly(éthylène-propylène) tels que ceux commercialisés sous la dénomination de « Kraton® » par la société SHELL CHEMICAL CO ou encore du type polystyrène/copoly(éthylène-butylène).

Parmi les gélifiants lipophiles pouvant être utilisés dans les compositions selon l'invention, on peut encore citer les esters de dextrine et d'acide gras, tels que les palmitates de dextrine, notamment tels que ceux commercialisés sous les dénominations de « Rheopearl TL® » ou « Rheopearl KL® » par la société CHIBA FLOUR.

Les gélifiants lipophiles peuvent être présents dans la composition selon l'invention en une teneur allant de 0,05 à 40% en poids par rapport au poids total de la composition, de préférence de 0,5 à 20% et mieux de 1 à 15% en poids.

Comme gélifiant hydrophile ou hydrosolubles, on peut citer :

- les homo- ou copolymères d'acides acrylique ou méthacrylique ou leurs sels et leurs esters et en particulier les produits vendus sous les dénominations « VERSICOL F »ou « VERSICOL K » par la société ALLIED COLLOID, « UTRAHOLD 8 » par la société CIBA-GEIGY, les acides polyacryliques de type SYNTHALEN K,
- les copolymères d'acide acrylique et d'acrylamide vendus sous la forme de leur sel de sodium sous les dénominations « RETEN » par la société HERCULES, le polyméthacrylate de sodium vendu sous la dénomination « DARVAN N°7 » par la société VANDERBILT, les sels de sodium d'acides polyhydroxycarboxyliques vendus sous la dénomination « HYDAGEN F » par la société HENKEL,
- les copolymères acide polyacryliques/acrylates d'alkyle de type PEMULEN,
- l'AMPS (Acide polyacrylamidométhyl propane sulfonique neutralisé partiellement à l'ammoniaque et hautement réticulé) commercialisé par la société CLARIANT,
- les copolymères AMPS/acrylamide de type SEPIGEL ou SIMULGEL commercialisés par la société SEPPIC, et
- les copolymères AMPS/méthacrylates d'alkyle polyoxyéthylénés (réticulés ou non) et leurs mélanges.

Comme autres exemples de polymères gélifiants hydrosolubles, on peut citer :

- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique;
- les polyuréthanes associatifs tels que le polymère $C_{16}$-$OE_{120}$-$C_{16}$ de la société SERVO DELDEN (commercialisé sous le nom SER AD FX1100, molécule à fonction uréthanne et poids moléculaire moyen en poids de 1300), OE étant un motif oxyéthyléné, le Rhéolate 205 à fonction urée vendu par la société RHEOX ou encore le Rhéolate 208 ou 204 (ces polymères étant vensus sous forme pure) ou le DW 1206B de chez RHOM & HAAS à chaîne alkyle en $C_{20}$ et à liaison uréthane, vendu à 20 % en matière sèche dans l'eau. On peut aussi utiliser des solutions ou dispersions de ces polyuréthanes associatifs notamment dans l'eau ou en milieu hydroalcoolique. A titre d'exemple, de tels polymères on peut citer, le SER AD FX1010, le SER AD FX1035 et le SER AD 1070 de la société SERVO DELDEN, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS, ou bien encore le Borchigel LW 44 de la société BORCHERS,
- les polymères d'origine naturelle, éventuellement modifiés, tels que :
  - les gommes arabiques, la gomme de guar, les dérivés du xanthane, la gomme de karaya ;
  - les alginates et les carraghénanes ;
  - les glycoaminoglycanes, l'acide hyaluronique et ses dérivés ;
  - la résine shellac, la gomme de sandaraque, les dammars, les élémis, les copals ;
  - l'acide désoxyribonucléïque ;
  - les muccopolysaccharides tels que l'acide hyaluronique, les chondroïtines sulfate, et leurs mélanges.

Les gélifiants hydrophiles peuvent être présents dans la composition selon l'invention en une teneur allant de 0,05 à 20% en poids par rapport au poids total de la composition, de préférence de 0,5 à 10% et mieux de 0,8 à 5% en poids.

La composition selon l'invention peut contenir des agents tensioactifs émulsionnants présents notamment en une proportion allant de 0,5 à 30 % en poids par rapport au poids total de la composition, mieux de 2 à 15% et mieux de 3 à 10 %. Ces agents tensioactifs peuvent être choisis parmi des agents tensioactifs anioniques, cationiques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis parmi :

a) les agents tensioactifs non ioniques de HLB supérieur ou égal à 8 à 25 °C, utilisés seuls ou en mélange; on peut citer notamment :

- les éthers oxyéthylénés et/ou oxypropylénés (pouvant comporter de 1 à 150 groupes oxyéthylénés et/ou oxypropylénés) de glycérol ;
- les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool cétéarylique à 30 groupes oxyéthylénés (nom CTFA "Ceteareth-30 ") et l'éther oxyéthyléné du mélange d'alcools gras en C12-C15 comportant 7 groupes oxyéthylénés (nom CTFA "C12-15 Pareth-7" commercialisé sous la dénomination « NEODOL 25-7 »® par SHELL CHEMICALS ;
- les esters d'acide gras (notamment d'acide en C8-C24, et de préférence en C16-C22) et de polyéthylène glycol (pouvant comprendre de 1 à 150 motifs d'éthylèneglycol) tels que le stéarate de PEG-50 et le monostéarate de PEG-40 commercialisé sous le nom MYRJ 52P par la société ICI UNIQUEMA;
- les esters d'acide gras (notamment d'acide en C8-C24, et de préférence en C16-C22) et des éthers de glycérol oxyéthylénés et/ou oxypropylénés (pouvant comporter de 1 à 150 groupes oxyéthylénés et/ou oxypropylénés), comme le monostéarate de PEG-200 glycéryle vendu sous la dénomination « Simulsol 220 TM » par la société SEPPIC ; le stéarate de glycéryle polyéthoxylé à 30 groupes d'oxyde d'éthylène comme le produit TAGAT S vendu par la société GOLDSCHMIDT, l'oléate de glycéryle polyéthoxylé à 30 groupes d'oxyde d'éthylène comme le produit TAGAT O vendu par la société GOLDSCHMIDT, le cocoate de glycéryle polyéthoxylé à 30 groupes d'oxyde d'éthylène comme le produit VARIONIC LI 13 vendu par la société SHEREX, l'isostéarate de glycéryle polyéthoxylé à 30 groupes d'oxyde d'éthylène comme le produit TAGAT L vendu par la société GOLDSCHMIDT et le laurate de glycéryle polyéthoxylé à 30 groupes d'oxyde d'éthylène comme le produit TAGAT I de la société GOLDSCHMIDT ;
- les esters d'acide gras (notamment d'acide en C8-C24, et de préférence en C16-C22) et des éthers de sorbitol oxyéthylénés et/ou oxypropylénés (pouvant comporter de 1 à 150 groupes oxyéthylénés et/ou oxypropylénés), comme le polysorbate 60 vendu sous la dénomination « Tween 60 » par la société UNIQUEMA ;
- la diméthicone copolyol, telle que celle vendue sous la dénomination « Q2-5220 » par la société DOW CORNING ;
- la diméthicone copolyol benzoate (FINSOLV SLB 101 et 201 de la société FINTEX) ;
- les copolymères d'oxyde propylène et d'oxyde d'éthylène, également appelés polycondensats OE/Opcomme par exemple les polycondensats tribloc polyéthylène glycol / polypropylène glycol / polyéthylène glycol vendus sous les dénominations "SYNPERONIC" comme les "SYNPERONIC PE/ L44" et "SYNPERONIC PE/F127 " par la société ICI, et leurs mélanges.
- et leurs mélanges.

b) les agents tensioactif non ioniques de HLB inférieur à 8 à 25 °C, éventuellement associés à un ou plusieurs agents tensioactif non ioniques de HLB supérieur à 8 à 25 °C, tels que cités ci-dessus tels que :

- les esters et éthers d'oses tels que les stéarate de sucrose, cocoate de sucrose, stéarate de sorbitan et leurs mélanges comme l'Arlatone 2121 commercialisé par la société ICI ;
- les esters d'acides gras (notamment d'acide en C8-C24, et de préférence en C16-C22) et de polyol, notamment de glycérol ou de sorbitol, tels que stéarate de glycéryle, stéarate de glycéryle tel que le produit vendu sous la dénomination TEGIN M par la société GOLDSCHMIDT, laurate de glycéryle tel que le produit vendu sous la dénomination IMWITOR 312 par la société HULS, stéarate de polyglycéryl-2, tristéarate de sorbitan, ricinoléate de glycéryle ;
- le mélange de cyclométhicone/diméthicone copolyol vendu sous la dénomination "Q2-3225C" par la société DOW CORNING.

c) Les tensioactifs anioniques tels que :

- les sels d'acides gras en $C_{16}$-$C_{30}$ notamment ceux dérivant des amines, comme le stéarate de triéthanolamine;
- les sels d'acides gras polyoxyéthylénés notamment ceux dérivant des amines ou les sels alcalins, et leurs mélanges ;
- les esters phosphoriques et leurs sels tels que le "DEA oleth-10 phosphate" (Crodafos N 10N de la société CRODA) ;
- les sulfosuccinates tels que le "Disodium PEG-5 citrate lauryl sulfosuccinate" et le "Disodium ricinoleamido MEA sulfosuccinate"
- les alkyléthersulfates tels que le lauryl éther sulfate de sodium ;
- les iséthionates ;
- les acylglutamates tels que le "Disodium hydrogenated tallow glutamate" (AMISOFT HS-21 R commercialisé par la société AJINOMOTO) et leurs mélanges. Convient tout particulièrement à l'invention, le stéarate de triéthanolamine. Ce dernier est généralement obtenu par simple mélange de l'acide stéarique et de la triéthanolamine.

On utilise de préférence des tensioactifs permettant l'obtention d'émulsion huile-dans-eau ou cire-dans-eau.
Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.
La composition selon l'invention peut comprendre un milieu aqueux, constituant une phase aqueuse, qui peut former la phase continue de la composition.
La phase aqueuse peut être constituée essentiellement d'eau ; elle peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$ et leur mélanges.
La phase aqueuse (eau et éventuellement le solvant miscible à l'eau) peut être présente, en une teneur allant de 0,1 % à 95 % en poids, par rapport au poids total de la composition, de préférence allant de 1 % à 80 % en poids
La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphasé ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de 200 $s^{-1}$, après 10 minutes de mesure en géométrie cône/plan). La composition peut être anhydre, par exemple il peut s'agir d'une pâte ou d'une stick anhydre. La composition peut être une composition non rinçée.
L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.
Selon un autre aspect, l'invention concerne également un ensemble cosmétique comprenant :

i) un récipient délimitant au moins un compartiment, ledit récipient étant fermé par un élément de fermeture ; et
ii) une composition disposée à l'intérieur dudit compartiment, la composition étant conforme à l'une quelconque des revendications qui précèdent.

Le récipient peut être sous toute forme adéquate. Il peut être notamment sous forme d'un flacon, d'un tube, d'un pot, d'un étui, d'une boite, d'un sachet ou d'un boîtier.
L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.
Le récipient peut être associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être sous forme d'un pinceau, tel que décrit par exemple dans le brevet FR 2 722 380. L'applicateur peut être sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US 5 492 426. L'applicateur peut

être solidaire du récipient, tel que décrit par exemple le brevet FR 2 761 959.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient. Alternativement, notamment lorsque le produit est sous forme d'un stick, ce dernier peut être entraîné par un mécanisme à piston. Toujours dans le cas d'un stick, notamment de produit de maquillage (rouge à lèvres, fond de teint, etc.), le récipient peut comporter un mécanisme, notamment à crémaillère, ou avec une tige filetée, ou avec une rampe hélicoïdale, et apte à déplacer un stick en direction de ladite ouverture. Un tel mécanisme est décrit par exemple dans le brevet FR 2 806 273 ou dans le brevet FR 2 775 566. Un tel mécanisme pour un produit liquide est décrit dans le brevet FR 2 727 609.

Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO 03/018423 ou dans le brevet FR 2 791 042.

Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

La composition peut être à la pression atmosphérique à l'intérieur du récipient (à température ambiante) ou pressurisée, notamment au moyen d'un gaz propulseur (aérosol). Dans ce dernier cas, le récipient est équipé d'une valve (du type de celles utilisées pour les aérosols).

Le contenu des brevets ou demandes de brevets cités précédemment sont incorporés par référence dans la présente demande.

L'invention est illustrée plus en détails par les exemples décrits ci-après.

## Exemples 1 et 2 : Préparation de dispersion de particules de polymère

Les exemples 1 et 2 illustrent la préparation de polymères conformes à l'invention, aptes à former une dispersion de particules dans un milieu organique considéré.

Dans ces exemples, on détermine, après préparation de ladite dispersion, les masses molaires moyennes en poids (Mw) et en nombre (Mn) du polymère, la température de transition vitreuse du polymère, le taux de matière sèche (ou extrait sec) de la dispersion et la taille des particules de polymères.

Les masses molaires moyennes en poids (Mw) et en nombre (Mn) sont déterminées par chromatographie liquide par perméation de gel (solvant THF, courbe d'étalonnage établie avec des étalons de polystyrène linéaire, détecteur réfractométrique).

La mesure de la température de transition vitreuse (Tg) est effectuée selon la norme ASTM D3418-97, par analyse enthalpique différentielle (DSC "Differential Scanning Calorimetry") sur calorimètre, sur une plage de température comprise entre -100°C et +150°C à une vitesse de chauffe de 10°C/min dans des creusets en aluminium de 150 μl. La préparation des creusets se fait de la manière suivante : dans un creuset en aluminium de 150 μl on introduit 100 μl de la dispersion obtenue et on laisse le solvant s'évaporer pendant 24h à température ambiante et à 50% d'humidité relative. On renouvelle l'opération puis on introduit le creuset dans le calorimètre Mettler DSC30.

Le taux de matière sèche (ou extrait sec), c'est à dire la teneur en matière non volatile, peut être mesurée de différentes manières : on peut citer par exemple les méthodes par séchage à l'étuve ou les méthodes par séchage par exposition à un rayonnement infrarouge.

De préférence, le taux de matière sèche de la dispersion de polymère obtenue est mesuré par échauffement de l'échantillon par des rayons infrarouges de 2 µm à 3,5 µm de longueur d'onde. Les substances contenues dans la composition qui possèdent une pression de vapeur élevée s'évaporent sous l'effet de ce rayonnement. La mesure de la perte de poids de l'échantillon permet de déterminer l'extrait sec de la composition. Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant: on étale environ 1 g de la dispersion sur une coupelle métallique. Celle-ci, après introduction dans le dessiccateur, est soumise à une consigne de température de 120°C pendant une heure. La masse humide de l'échantillon, correspondant à la masse initiale et la masse sèche de l'échantillon, correspondant à la masse après exposition au rayonnement, sont mesurées au moyen d'une balance de précision.

Le taux de matière sèche est calculée de la manière suivante :

$$\text{Extrait Sec} = 100 \times (\text{masse sèche} / \text{masse humide}).$$

Les tailles de particules peuvent être mesurées par différentes techniques : on peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique. De préférence, les tailles et les distributions de tailles des particules des compositions selon l'invention, sont mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.

La composition est caractérisée par son diamètre « effectif » moyen en volume D[4,3], défini de la manière suivante :

$$D[4,3] = \frac{\sum_i V_i \cdot d_i}{\sum_i V_i}$$

où $V_i$ représente le volume des particules de diamètre effectif $d_i$. Ce paramètre est notamment décrit dans la documentation technique du granulomètre.

Les mesures sont réalisées à 25 °C, sur une dispersion de particules diluée, obtenue à partir de la composition de la manière suivante : 1) dilution d'un facteur 100 avec de l'eau, 2) homogénéisation de la solution, 3) repos de la solution durant 18 heures, 4) récupération du surnageant homogène blanchâtre.

Le diamètre « effectif » est obtenu en prenant un indice de réfraction de 1,33 pour l'eau et un indice de réfraction moyen de 1,42 pour les particules.

**Exemple 1**

Cet exemple illustre la préparation d'une dispersion de particules d'un polymère dans l'isododécane, ledit polymère étant obtenu par polymérisation d'acrylate de méthyle et le macromonomère correspondant à un copolymère polyéthylène/polybutylène à groupement terminal méthacrylate (Kraton L-1253).

Dans un réacteur, on charge 2 kg d'heptane, 2 kg d'isododécane, 2,8 kg d'acrylate de méthyle et 1,2 kgg de macromonomère du type copolymère de polyéthylène/polybutylène à groupement terminal méthacrylate (Kraton L-1253) et 320 g de tertio butyl peroxy-2-éthylhexanoate (Trigonox 21 S).

On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 16 kg d'acrylate de méthyle et 200 g de Trigonox 21S.

On laisse ensuite le chauffage pendant 4 heures à 90°C puis on distille l'heptane du milieu réactionnel. A l'issue de cette opération de distillation, on obtient une dispersion de particules de polymère ainsi préparé stable dans l'isododécane

Le polymère greffé comprend 6% en poids de macromonomère par rapport au poids du polymère.

Les caractéristiques du polymère et des particules formées par ledit polymère sont les suivantes :

- Masse moléculaire poids Mw = 119900
- Masse moléculaire nombre Mn = 16300
- Indice de polydispersité (Mw/Mn) = 7.37
- Transition vitreuse : 10°C par DSC Mettler ;
- Extrait sec : 52.4 % dans l'isododécane, réalisé par thermobalance ;
- Granulométrie : 46 nm avec polydispersité de 0,05 réalisée sur Malvern Autosizer Lo-C à 25°C

La stabilité de la dispersion obtenue est mise en évidence par la mise en oeuvre du protocole de stabilité suivant : dans un tube à hémolyse, on place 8 ml de la dispersion réalisée et on centrifuge à 4000 tours/min pendant 15 minutes à l'aide d'une centrifugeuse Jouan C100-S5. Au bout de 15 minutes, on constate qu'il n'y a pas de déphasage ce qui démontre que la dispersion est stable.

**Exemple 2**

Cet exemple illustre la préparation d'un polymère formant une dispersion de particules dans un solvant carboné, ledit polymère étant obtenu par polymérisation d'acrylate de méthyle, d'acide acrylique et le macromonomère correspondant à un copolymère polyéthylène/polybutylène à groupement terminal méthacrylate (Kraton L-1253).

Dans un réacteur de 1 litre, on charge 200 g d'heptane, 200 g d'isododécane, 28 g d'acrylate de méthyle et 12 g de macromonomère du type copolymère de polyéthylène/polybutylène à groupement terminal méthacrylate (Kraton L-1253) et 3.2 g de tertio butyl peroxy-2-éthylhexanoate (Trigonox 21 S).

On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 150 g d'acrylate de méthyle, 10g d'acide acrylique et 2 g de Trigonox 21 S.

On laisse ensuite le chauffage pendant 4 heures à 90°C puis on distille l'heptane du milieu réactionnel. A l'issue de cette opération de distillation, on obtient une dispersion de particules de polymère ainsi préparé stable dans l'isododécane

Le polymère greffé comprend 6 % en poids de macromonomère par rapport au poids du polymère.

Les caractéristiques du polymère et des particules formées par ledit polymère sont les suivantes :

- Masse moléculaire poids Mw=143639
- Masse moléculaire nombre Mn=23965
- Indice de polydispersité (Mw/Mn)= 5.99
- Extrait sec théorique : 51.3 % dans l'isododécane
- Granulométrie : 48 nm avec polydispersité de 0,04 réalisée sur Malvern Autosizer Lo-C à 25°C ;

Après la mise en oeuvre du protocole de stabilité conformément à l'exemple 1, on constate que la dispersion obtenue est stable.

**Exemple 3 : Stick de rouge à lèvres**

| Ingrédients Nom INCI | % massique |
|---|---|
| Octyldodecanol | 11,75 |
| VP eicosene copolymer | 15 |
| Polyethylène (cire de polyéthylène PM 500) | 13 |
| Pigments | 10,25 |
| Dispersion de polymère de l'exemple 1 | 50 |

Dans un poêlon, on introduit la cire, la phase huileuse et les pigments sous la forme d'un broyat dans la phase huileuse contenant la PVP eicosene. Le mélange est mis à fondre à 100°C sous agitation Rayneri. Lorsque la préparation est liquide, on laisse l'ensemble à 100°C pendant 40 minutes. On introduit alors les ingrédients volatils ou contenant

des solvants volatils. Le poêlon est couvert pour limiter les évaporations et le mélange est laissé sous agitation pendant 10 minutes. La formule est alors coulée à 42°C avant d'être placée au congélateur. On démoule lorsque la température du moule est d'environ 4°C.

Le transfert de cette formule a été mesuré selon le protocole décrit précédemment. Il est égal à 1,85 ± 0,1.

**Exemple 4 : Stick de rouge à lèvres**

| Ingrédients | % massique |
|---|---|
| Pigments | 8,20 |
| Hydrogenated polyisobutene (Parleam) | 5,18 |
| Polyhydroxystearic acid | 0,21 |
| C30-C50 alcohols | 2 |
| Polyethylene (cire de polyéthylène PM 500) | 10 |
| Sucrose acetate isobutyrate | 5 |
| Dispersion de polymère de l'exemple 1 | 68,82 |
| Parfum | Qsp 100 |

Mode opératoire

Dans un poêlon, sous agitation Rayneri, on introduit les cires, les pâtes pigmentaires et l'ester de saccharose, on se place à 105°C, on laisse tourner 30 minutes, puis on ajoute les nacres, on ajoute ensuite la dispersion de polymère et le parfum, on laisse tourner 10 minutes, puis on coule dans un moule à 42°C. On place le moule au congélateur et on démoule lorsque le moule est à environ 4°C.

Le transfert mesuré selon le protocole décrit précédemment est égal à 1,2.

**Exemple 5 : Stick de rouge à lèvres**

| Ingrédients Nom INCI | % massique |
|---|---|
| Octyldodecanol | 11,75 |
| VP eicosene copolymer | 15 |
| Polyethylène (cire de polyéthylène PM 500) | 13 |
| Pigments | 10,25 |
| Dispersion de polymère de l'exemple 2 | 50 |

Cette composition a été préparée selon le même mode opératoire que celui de l'exemple 1.

**Exemple 6 : Mascara**

| Phase A | |
|---|---|
| Cire de Candellila | 15% |
| Acide stéarique | 5,8 % |
| Dispersion de polymère de l'exemple 2 | 10% |

(suite)

| Phase B | |
|---|---|
| Triéthanolamine | 2,9 % |
| Hydroxyéthylcellulose | 0,9 % |
| Gomme arabique | 3,5 % |
| Oxyde de fer noir | 8% |
| Conservateurs | Qs % |
| Eau | Qsp 100 |

Mode opératoire :

Cette composition peut être préparée de manière standard par formation à chaud d'une émulsion cire dans eau.

On chauffe la phase grasse (phase A) contenant la cire et l'acide stéarique jusqu'à fusion complète de l'ensemble des constituants. Ensuite, la dispersion de polymère de l'exemple 2 et les pigments sont incorporés sous agitation à la phase huileuse. Parallèlement, la phase aqueuse (phase B) contenant le neutralisant (Triéthanolamine), les polymères gélifiants sont portés à une température au moins égale à la température de la phase grasse. La phase aqueuse est ensuite additionnée sur la phase huileuse, sous forte agitation (3000rpm), pour former l'émulsion à chaud. L'agitation, la température sont maintenues durant environ 30 minutes.

Une agitation modérée à la pâle est ensuite appliquée jusqu'à retour à la température ambiante.

## Revendications

1. Composition cosmétique comprenant, dans un milieu cosmétiquement acceptable, une dispersion de particules d'un polymère acrylique greffé non siliconé dans une phase grasse liquide,
ledit polymère acrylique greffé étant un polymère susceptible d'être obtenu par polymérisation radicalaire :

   - d'un ou plusieurs monomère(s) acrylique(s) comprenant au moins l'acide (méth)acrylique et au moins un monomère choisi parmi les (méth)acrylates d'alkyle en $C_1$-$C_3$,
   - avec le méthacrylate de poly(éthylène/butylène) comme macromonomère(s) carboné ayant une masse moléculaire moyenne en poids supérieure ou égale à 200 et la teneur en macromonomère polymérisé représentant de 0,05 à 20% en poids du polymère , dans un milieu organique de polymérisation.

2. Composition selon l'une des revendications précédentes, **caractérisée par le fait que** l'acide (méth)acrylique est présent en une teneur d'au moins 5 % en poids, par rapport au poids total du polymère, notamment allant de 5 % à 80 % en poids, de préférence d'au moins 10 % en poids, notamment allant de 10 % en poids à 70 % en poids, préférentiellement d'au moins 15 % en poids, notamment allant de 15 % à 60 % en poids.

3. Composition selon l'une des revendications précédentes, **caractérisée par le fait que** le polymère acrylique greffé ne contient pas de monomère vinylique non acrylique additionnel.

4. Composition selon l'une quelconque des revendications précédentes, **caractérisée en ce que** le macromonomère a une masse moléculaire moyenne en poids supérieure ou égale à 300, préférentiellement supérieure ou égale à 500, et plus préférentiellement supérieure à 600.

5. Composition selon l'une quelconque des revendications précédentes, **caractérisée en ce que** le macromonomère a une masse moléculaire moyenne en poids (Mw) allant de 300 à 100 000, de préférence allant de 500 à 50 000, préférentiellement allant de 800 à 20 000, plus préférentiellement allant de 800 à 10000, et encore plus préférentiellement allant de 800 à 6000.

6. Composition selon l'une quelconque des revendications précédentes; **caractérisée en ce que** le macromonomère carboné polymérisé représente de 0,1 à 15 % en poids du poids total du polymère, de préférence de 0,2 à 10 % en poids, et préférentiellement de 0,3 à 8 % en poids.

**7.** Composition selon l'une des revendications précédentes, **caractérisée en ce que** le polymère acrylique greffé a une masse moléculaire moyenne en poids (Mw) comprise entre 10 000 et 300 000, notamment entre 20 000 et 200 000, mieux encore entre 25 000 et 150 000.

**8.** Composition selon l'une des revendications précédentes, **caractérisée en ce que** particules de polymère éthylénique greffé ont une taille moyenne allant de 10 à 400 nm, de préférence allant de 20 à 200 nm.

**9.** Composition selon l'une des revendications précédentes, **caractérisée en ce que** le polymère éthylénique greffé est un polymère filmogène.

**10.** Composition selon l'une des revendications précédentes, **caractérisée par le fait que** le polymère greffé non siliconé contient majoritairement un macromonomère carboné et contient éventuellement au plus 7 % en poids de macromonomère siliconé.

**11.** Composition selon l'une des revendications précédentes, **caractérisée par le fait que** le polymère greffé non siliconé est exempt de macromonomère siliconé.

**12.** Composition selon l'une des revendications précédentes, **caractérisée en ce que** le polymère éthylénique greffé non siliconé comprend un squelette éthylénique insoluble dans ladite phase grasse liquide et des chaînes latérales liées de manière covalente audit squelette et solubles dans ladite phase grasse liquide.

**13.** Composition selon l'une des revendications précédentes, **caractérisée en ce que** le polymère éthylénique est dispersé en l'absence de stabilisant additionnel en surface des particules.

**14.** Composition selon l'une des revendications précédentes, **caractérisée en ce que** le polymère éthylénique greffé est présent en une teneur en matière sèche allant de 1 à 70% en poids par rapport au poids total de la composition, mieux de 5 à 60% en poids, de préférence allant de 6 à 45% et mieux allant de 8 à 40% en poids.

**15.** Composition selon l'une des revendications précédentes, **caractérisée en ce que** la phase grasse liquide comprend un composé organique liquide choisi parmi :

- les composés organiques liquides ayant un paramètre de solubilité global selon l'espace de solubilité de Hansen inférieur ou égal à 18 $(MPa)^{1/2}$,
- les monoalcools ayant un paramètre de solubilité global selon l'espace de solubilité de Hansen inférieur ou égal à 20 $(MPa)^{1/2}$ ; et
- leurs mélanges.

**16.** Composition selon l'une quelconque des revendications précédentes, **caractérisée par le fait qu'**elle comprend une huile volatile choisie parmi l'isododécane, l'isodécane, l'isohexadécane.

**17.** Composition selon la revendication précédente, **caractérisée par le fait que** l'huile volatile est présente en une teneur allant de 1 % à 70 % en poids, par rapport au poids total de la composition, de préférence allant de 5 % à 50 % en poids, et préférentiellement allant de 10 % à 35 % en poids.

**18.** Composition selon l'une des revendications précédentes, **caractérisée en ce que** la phase grasse liquide est une phase grasse liquide non siliconée ne contenant pas de composés organiques liquides siliconés.

**19.** Composition selon l'une quelconque des revendications précédentes, **caractérisée par le fait qu'**elle comprend au moins un corps gras solides à température ambiante choisi parmi les cires, les corps gras pâteux, les gommes et leurs mélanges.

**20.** Composition selon l'une quelconque des revendications précédentes, **caractérisée par le fait qu'**elle comprend une matière colorante.

**21.** Composition selon l'une quelconque des revendications précédentes, **caractérisée par le fait qu'**elle comprend un ingrédient cosmétique choisi parmi les polymères filmogènes additionnels, les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les fibres, les agents anti-chutes des cheveux, les agents de soin du cil, les agents antipelliculaires, les agents propulseurs, ou leurs mélanges.

**22.** Composition cosmétique selon l'une quelconque des revendications précédentes, **caractérisée par le fait qu'**elle est un rouge à lèvres ou un mascara.

**23.** Procédé cosmétique de maquillage ou de soin non thérapeutique des matières kératiniques, en particulier de la peau ou des lèvres, comprenant l'application sur les matières kératiniques, en particulier de la peau ou des lèvres, d'une composition selon l'une quelconque des revendications 1 à 22.

**24.** Procédé cosmétique de maquillage ou de soin non thérapeutique des fibres kératiniques, en particulier des cils comprenant l'application sur les fibres kératiniques, en particulier les cils, d'une composition selon l'une quelconque des revendications 1 à 22.

## Patentansprüche

**1.** Kosmetische Zusammensetzung, die in einem kosmetisch unbedenklichen Medium eine Dispersion von Teilchen aus einem nicht auf Silikon basierenden gepfropften Acrylpolymer in einer flüssigen Fettphase umfasst,
wobei es sich bei dem gepfropften Acrylpolymer um ein Polymer handelt, das durch radikalische Polymerisation:

- von einem oder mehreren Acrylmonomeren, die mindestens (Meth)acrylsäure und mindestens ein Monomer, das aus $C_1$-$C_3$-Alkyl(meth)acrylaten ausgewählt ist, umfassen,
- mit Poly(ethylen/butylen)methacrylat als auf Kohlenstoff basierendes Makromonomer bzw. auf Kohlenstoff basierende Makromonomere mit einem gewichtsmittleren Molekulargewicht größer gleich 200, wobei der Gehalt an polymerisiertem Makromonomer 0,05 bis 20 Gew.-% des Polymers, beträgt,

in einem organischen Polymerisationsmedium erhältlich ist.

**2.** Zusammensetzung nach einem der vorhergehenden Ansprüche, **dadurch gekennzeichnet, dass** die (Meth)acrylsäure in einem Gehalt von mindestens 5 Gew.-%, bezogen auf das Gesamtgewicht des Polymers, insbesondere im Bereich von 5 bis 80 Gew.-%, vorzugsweise mindestens 10 Gew.-%, insbesondere im Bereich von 10 Gew.-% bis 70 Gew.-%, bevorzugt mindestens 15 Gew.-%, insbesondere im Bereich von 15 bis 60 Gew.-%, vorliegt.

**3.** Zusammensetzung nach einem der vorhergehenden Ansprüche, **dadurch gekennzeichnet, dass** das gepfropfte Acrylpolymer kein zusätzliches Nicht-Acryl-Vinylmonomer enthält.

**4.** Zusammensetzung nach einem der vorhergehenden Ansprüche, **dadurch gekennzeichnet, dass** das Makromonomer ein gewichtsmittleres Molekulargewicht größer gleich 300, bevorzugt größer gleich 500 und noch weiter bevorzugt größer 600 aufweist.

**5.** Zusammensetzung nach einem der vorhergehenden Ansprüche, **dadurch gekennzeichnet, dass** das Makromonomer ein gewichtsmittleres Molekulargewicht (Mw) im Bereich von 300 bis 100.000, vorzugsweise im Bereich von 500 bis 50.000, bevorzugt im Bereich von 800 bis 20.000, weiter bevorzugt im Bereich von 800 bis 10.000 und noch weiter bevorzugt im Bereich von 800 bis 6000 aufweist.

**6.** Zusammensetzung nach einem der vorhergehenden Ansprüche, **dadurch gekennzeichnet, dass** das polymerisierte auf Kohlenstoff basierende Makromonomer 0,1 bis 15 Gew.-% des Gesamtgewichts des Polymers, vorzugsweise 0,2 bis 10 Gew.-% und bevorzugt 0,3 bis 8 Gew.-% ausmacht.

**7.** Zusammensetzung nach einem der vorhergehenden Ansprüche, **dadurch gekennzeichnet, dass** das gepfropfte Acrylpolymer ein gewichtsmittleres Molekulargewicht (Mw) zwischen 10.000 und 300.000, insbesondere zwischen 20.000 und 200.000 und noch besser zwischen 25.000 und 150.000 aufweist.

**8.** Zusammensetzung nach einem der vorhergehenden Ansprüche, **dadurch gekennzeichnet, dass** Teilchen aus gepfropftem ethylenischem Polymer eine mittlere Größe im Bereich von 10 bis 400 nm und bevorzugt im Bereich von 20 bis 200 nm aufweisen.

**9.** Zusammensetzung nach einem der vorhergehenden Ansprüche, **dadurch gekennzeichnet, dass** es sich bei dem

gepfropften ethylenischen Polymer um ein filmbildendes Polymer handelt.

**10.** Zusammensetzung nach einem der vorhergehenden Ansprüche, **dadurch gekennzeichnet, dass** das nicht auf Silikon basierende gepfropfte Polymer hauptsächlich ein auf Kohlenstoff basierendes Makromonomer enthält und gegebenenfalls höchstens 7 Gew.-% auf Silikon basierendes Makromonomer enthält.

**11.** Zusammensetzung nach einem der vorhergehenden Ansprüche, **dadurch gekennzeichnet, dass** das nicht auf Silikon basierende gepfropfte Polymer frei von auf Silikon basierendem Makromonomer ist.

**12.** Zusammensetzung nach einem der vorhergehenden Ansprüche, **dadurch gekennzeichnet, dass** das nicht auf Silikon basierende gepfropfte ethylenische Polymer eine ethylenische Hauptkette, die in der flüssigen Fettphase unlöslich ist, und Seitenketten, die kovalent an die Hauptkette gebunden sind und in der flüssigen Fettphase löslich sind, umfasst.

**13.** Zusammensetzung nach einem der vorhergehenden Ansprüche, **dadurch gekennzeichnet, dass** das ethylenische Polymer in Abwesenheit von zusätzlichem Stabilisator an der Oberfläche der Teilchen dispergiert ist.

**14.** Zusammensetzung nach einem der vorhergehenden Ansprüche, **dadurch gekennzeichnet, dass** das gepfropfte ethylenische Polymer in einem Feststoffgehalt von 1 bis 70 Gew.-%, bezogen auf das Gesamtgewicht der zusammensetzung, besser von 5 bis 60 Gew.-%, bevorzugt im Bereich von 6 bis 45 Gew.-% und besser im Bereich von 8 bis 40 Gew.-% vorliegt.

**15.** Zusammensetzung nach einem der vorhergehenden Ansprüche, **dadurch gekennzeichnet, dass** die flüssige Fettphase eine flüssige organische Verbindung, die aus:

- flüssigen organischen Verbindungen mit einem globalen Löslichkeitsparameter gemäß dem Löslichkeitsraum nach Hansen kleiner gleich 18 $(MPa)^{1/2}$,
- Monoalkoholen mit einem globalen Löslichkeitsparameter gemäß dem Löslichkeitsraum nach Hansen kleiner gleich 20 $(MPa)^{1/2}$ und
- Mischungen davon

ausgewählt ist, enthält.

**16.** Zusammensetzung nach einem der vorhergehenden Ansprüche, **dadurch gekennzeichnet, dass** sie ein flüchtiges Öl, das aus Isododecan, Isodecan und Isohexadecan ausgewählt ist, umfasst.

**17.** Zusammensetzung nach dem vorhergehenden Anspruch, **dadurch gekennzeichnet, dass** das flüchtige Öl in einem Gehalt im Bereich von 1 bis 70 Gew.-%, bezogen auf das Gesamtgewicht der Zusammensetzung, vorzugsweise im Bereich von 5 bis 50 Gew.-% und bevorzugt im Bereich von 10 bis 35 Gew.-% vorliegt.

**18.** Zusammensetzung nach einem der vorhergehenden Ansprüche, **dadurch gekennzeichnet, dass** es sich bei der flüssigen Fettphase um eine nicht auf Silikon basierende flüssige Fettphase, die keine auf Silikon basierenden flüssigen organischen Verbindungen umfasst, handelt.

**19.** Zusammensetzung nach einem der vorhergehenden Ansprüche, **dadurch gekennzeichnet, dass** sie mindestens eine bei Umgebungstemperatur feste Fettsubstanz, die aus Wachsen, pastösen Fettsubstanzen, Gummen und Mischungen davon ausgewählt ist, umfasst.

**20.** zusammensetzung nach einem der vorhergehenden Ansprüche, **dadurch gekennzeichnet, dass** sie ein Farbmittel umfasst.

**21.** Zusammensetzung nach einem der vorhergehenden Ansprüche, **dadurch gekennzeichnet, dass** sie einen kosmetischen Bestandteil, der aus zusätzlichen filmbildenden Polymeren, Vitaminen, Verdickungsmitteln, Geliermitteln, Spurenelementen, zartmachenden Mitteln, Sequestriermitteln, Duft-Stoffen, Alkalinisierungsmitteln, Ansäuerungsmitteln, Konservierungsmitteln, Sonnenschutzfiltern, Tensiden, Antioxidantien, Fasern, Mitteln gegen Haarausfall, Wimpernpflegemitteln, Mitteln gegen Schuppen, Treibmitteln oder Mischungen davon ausgewählt ist, umfasst.

**22.** Kosmetische Zusammensetzung nach einem der vorhergehenden Ansprüche, **dadurch gekennzeichnet, dass** es

sich um einen Lippenstift oder eine Mascara handelt.

**23.** Nichttherapeutisches kosmetisches Verfahren zum Schminken oder zur Pflege von Keratinmaterialien, insbesondere der Haut oder der Lippen, bei dem man auf die Keratinmaterialien, insbesondere die Haut oder die Lippen, eine Zusammensetzung nach einem der Ansprüche 1 bis 22 aufbringt.

**24.** Nichttherapeutisches kosmetisches Verfahren zum Schminken oder zur Pflege von Keratinfasern, insbesondere der Wimpern, bei dem man auf die Keratinfasern, insbesondere die Wimpern, eine Zusammensetzung nach einem der Ansprüche 1 bis 22 aufbringt.

## Claims

**1.** Cosmetic composition comprising, in a cosmetically acceptable medium, a dispersion of particles of a non-silicone-based grafted acrylic polymer in a liquid fatty phase,
said grafted acrylic polymer being a polymer able to be obtained by radical polymerization:

- of one or more acrylic monomer(s) comprising at least (meth)acrylic acid and at least one monomer chosen from $C_1$-$C_3$ alkyl (meth) acrylates,
- with poly(ethylene/butylene) methacrylate as carbon-based macromonomer(s) having a weight-average molecular weight greater than or equal to 200 and the content of polymerized macromonomer representing from 0.05 to 20% by weight of the polymer, in an organic polymerization medium.

**2.** Composition according to one of the preceding claims, **characterized in that** the (meth)acrylic acid is present at an amount of at least 5% by weight, especially ranging from 5% to 80% by weight, preferably of at least 10% by weight, especially ranging from 10% by weight to 70% by weight, preferentially of at least 15% by weight, especially ranging from 15% to 60% by weight, relative to the total weight of the polymer.

**3.** Composition according to one of the preceding claims, **characterized in that** the grafted acrylic polymer does not contain additional non-acrylic vinyl monomer.

**4.** Composition according to any one of the preceding claims, **characterized in that** the macromonomer has a weight-average molecular weight greater than or equal to 300, preferentially greater than or equal to 500, and more preferentially greater than 600.

**5.** Composition according to any one of the preceding claims, **characterized in that** the macromonomer has a weight-average molecular weight (Mw) ranging from 300 to 100 000, preferably ranging from 500 to 50 000, preferentially ranging from 800 to 20 000, more preferentially ranging from 800 to 10 000, and even more preferentially ranging from 800 to 6000.

**6.** Composition according to any one of the preceding claims, **characterized in that** the polymerized carbon-based macromonomer represents from 0.1 to 15% by weight, preferably from 0.2 to 10% by weight, and preferentially from 0.3 to 8% by weight of the total weight of the polymer.

**7.** Composition according to one of the preceding claims, **characterized in that** the grafted acrylic polymer has a weight-average molecular weight (Mw) of between 10 000 and 300 000, especially between 20 000 and 200 000, better still between 25 000 and 150 000.

**8.** Composition according to one of the preceding claims, **characterized in that** particles of grafted ethylenic polymer have an average size ranging from 10 to 400 nm, preferably ranging from 20 to 200 nm.

**9.** Composition according to one of the preceding claims, **characterized in that** the grafted ethylenic polymer is a film-forming polymer.

**10.** Composition according to one of the preceding claims, **characterized in that** the non-silicone-based grafted polymer predominantly contains a carbon-based macromonomer and optionally contains at most 7% by weight of silicone-based macromonomer.

**11.** Composition according to one of the preceding claims, **characterized in that** the non-silicone-based grafted polymer does not contain silicone-based macromonomer.

**12.** Composition according to one of the preceding claims, **characterized in that** the non-silicone-based grafted ethylenic polymer comprises an ethylenic backbone that is insoluble in said liquid fatty phase and side chains covalently bonded to said backbone that are soluble in said liquid fatty phase.

**13.** Composition according to one of the preceding claims, **characterized in that** the ethylenic polymer is dispersed in the absence of additional stabilizer at the surface of the particles.

**14.** Composition according to one of the preceding claims, **characterized in that** the grafted ethylenic polymer is present at a solids content ranging from 1 to 70% by weight, better still from 5 to 60% by weight, preferably ranging from 6 to 45% and better still ranging from 8 to 40% by weight relative to the total weight of the composition.

**15.** Composition according to one of the preceding claims, **characterized in that** the liquid fatty phase comprises a liquid organic compound chosen from:

- liquid organic compounds having a global solubility parameter according to the Hansen solubility space of less than or equal to 18 $(MPa)^{1/2}$,
- monoalcohols having a global solubility parameter according to the Hansen solubility space of less than or equal to 20 $(MPa)^{1/2}$; and
- mixtures thereof.

**16.** Composition according to any one of the preceding claims, **characterized in that** it comprises a volatile oil chosen from isododecane, isodecane and isohexadecane.

**17.** Composition according to the preceding claim, **characterized in that** the volatile oil is present at a content ranging from 1% to 70% by weight, preferably ranging from 5% to 50% by weight, and preferentially ranging from 10% to 35% by weight, relative to the total weight of the composition.

**18.** Composition according to one of the preceding claims, **characterized in that** the liquid fatty phase is a non-silicone-based liquid fatty phase that does not contain silicone-based liquid organic compounds.

**19.** Composition according to any one of the preceding claims, **characterized in that** it comprises at least one fatty substance that is solid at room temperature, chosen from waxes, pasty fatty substances, gums and mixtures thereof.

**20.** Composition according to any one of the preceding claims, **characterized in that** it comprises a colorant.

**21.** Composition according to any one of the preceding claims, **characterized in that** it comprises a cosmetic ingredient chosen from additional film-forming polymers, vitamins, thickeners, gelling agents, trace elements, softeners, sequestering agents, fragrances, basifying or acidifying agents, preserving agents, sunscreens, surfactants, antioxidants, fibres, agents for preventing hair loss, eyelash care agents, antidandruff agents and propellants, or mixtures thereof.

**22.** Cosmetic composition according to any one of the preceding claims, **characterized in that** it is a lipstick or a mascara.

**23.** Non-therapeutic cosmetic process for making up or caring for keratin materials, in particular the skin or the lips, comprising the application, to the keratin materials, in particular the skin or the lips, of a composition according to any one of Claims 1 to 22.

**24.** Non-therapeutic cosmetic process for making up or caring for keratin fibres, in particular the eyelashes, comprising the application, to the keratin fibres, in particular the eyelashes, of a composition according to any one of Claims 1 to 22.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9717057 A
- EP 0923928 A
- EP 749747 A
- EP 895467 A
- EP 96459 A
- US 5625005 A
- FR 2792190 A
- US 4887622 A
- FR 2796529
- FR 2722380
- US 5492426 A
- FR 2761959
- WO 0103538 A
- FR 2806273
- FR 2775566
- FR 2727609
- WO 03018423 A
- FR 2791042
- FR 2792618


### Littérature non-brevet citée dans la description


- Solubility parameter values. **ERIC A.GRULKE.** Polymer Handbook. 519-559
- **C.M.HANSEN.** The three dimensional solubility parameters. *J.Paint Technol.,* 1967, vol. 39, 105
- **GILLMAN K.F.** *Polymer Letters,* 1967, vol. 5, 477-481
- Microemulsions Theory and Practice. Academic Press, 1977, 21-32
- CTFA. 1995
- Encyclopedia of Chemical Technology, KIRK-OTHMER. WILEY, 1979, vol. 22, 333-432
- **VAN DE HULST, H.C.** Light Scattering by Small Particles. Wiley, 1957